# EUROPEAN PATENT SPECIFICATION

(11) **EP 2 125 852 B1**
(45) Date of publication and mention of the grant of the patent: **06.04.2016**
(21) Application number: 08729961.6
(22) Date of filing: 15.02.2008
(51) Int. Cl.: C07H 19/06, C07H 19/16, C07H 21/00, C12N 15/11

(54) **5'-SUBSTITUTED-2'-F MODIFIED NUCLEOSIDES AND OLIGOMERIC COMPOUNDS PREPARED THEREFROM**
5'-SUBSTITUIERTE 2'-F-MODIFIZIERTE NUKLEOSIDE UND DARAUS HERGESTELLTE OLIGOMERE VERBINDUNGEN
NUCLÉOSIDES MODIFIÉS 5'-SUBSTITUÉS-2'-F ET COMPOSÉS OLIGOMÈRES PRÉPARÉS À PARTIR DE CEUX-CI

(30) Priority: 15.02.2007 US 890079 P
(43) Date of publication of application: 02.12.2009
(73) Proprietor: Ionis Pharmaceuticals, Inc., Carlsbad, CA 92010 (US)
(72) Inventor: MIGAWA, Michael, T., Carlsbad, CA 92011 (US); SWAYZE, Eric, E., Carlsbad, CA 92009 (US); BHAT, Balkrishen, Carlsbad, CA 92009 (US)
(74) Representative: Hallybone, Huw George
(86) International application number: PCT/US2008/054074
(87) International publication number: WO 2008/101157

(56) References cited:
- WO-A-94/22890
- US-A- 5 670 633

## Description

### FIELD OF THE INVENTION

The present invention provides 5'-substituted-2'-F modified nucleosides and oligomeric compounds prepared therefrom. More particularly, the present invention provides 5'-substituted-2'-F modified nucleosides, oligomeric compounds comprising at least one of these modified nucleosides and compositions comprising at least one of these oligomeric compounds. In some embodiments, the oligomeric compounds comprising at least one of the 5'-substituted-2'-F modified nucleosides hybridize to a portion of a target RNA resulting in loss of normal function of the target RNA.

### BACKGROUND OF THE INVENTION

Targeting disease-causing gene sequences was first suggested more than thirty years ago (Belikova et al., Tet. Lett., 1967, 37, 3557-3562), and antisense activity was demonstrated in cell culture more than a decade later (Zamecnik et al., Proc. Natl. Acad. Sci. U.S.A., 1978, 75, 280-284). One advantage of antisense technology in the treatment of a disease or condition that stems from a disease-causing gene is that it is a direct genetic approach that has the ability to modulate (increase or decrease) the expression of specific disease-causing genes. Another advantage is that validation of a therapeutic target using antisense compounds results in direct and immediate discovery of the drug candidate; the antisense compound is the potential therapeutic agent.

Generally, the principle behind antisense technology is that an antisense compound hybridizes to a target nucleic acid and modulates gene expression activities or function, such as transcription or translation. The modulation of gene expression can be achieved by, for example, target degradation or occupancy-based inhibition. An example of modulation of RNA target function by degradation is RNase H-based degradation of the target RNA upon hybridization with a DNA-like antisense compound. Another example of modulation of gene expression by target degradation is RNA interference (RNAi). RNAi generally refers to antisense-mediated gene silencing involving the introduction of dsRNA leading to the sequence-specific reduction of targeted endogenous mRNA levels. Regardless of the specific mechanism, this sequence-specificity makes antisense compounds extremely attractive as tools for target validation and gene functionalization, as well as therapeutics to selectively modulate the expression of genes involved in the pathogenesis of malignancies and other diseases.

Antisense technology is an effective means for reducing the expression of one or more specific gene products and can therefore prove to be uniquely useful in a number of therapeutic, diagnostic, and research applications. Chemically modified nucleosides are routinely used for incorporation into antisense compounds to enhance one or more properties, such as nuclease resistance, pharmacokinetics or affinity for a target RNA. In 1998, the antisense compound, Vitravene® (fomivirsen; developed by Isis Pharmaceuticals Inc., Carlsbad, CA) was the first antisense drug to achieve marketing clearance from the U.S. Food and Drug Administration (FDA), and is currently a treatment of cytomegalovirus (CMV)-induced retinitis in AIDS patients.

New chemical modifications have improved the potency and efficacy of antisense compounds, uncovering the potential for oral delivery as well as enhancing subcutaneous administration, decreasing potential for side effects, and leading to improvements in patient convenience. Chemical modifications increasing potency of antisense compounds allow administration of lower doses, which reduces the potential for toxicity, as well as decreasing overall cost of therapy. Modifications increasing the resistance to degradation result in slower clearance from the body, allowing for less frequent dosing. Different types of chemical modifications can be combined in one compound to further optimize the compound's efficacy.

The synthesis of 5'-substituted DNA and RNA derivatives and their incorporation into oligomeric compounds has been reported in the literature (Saha et al., J. Org. Chem., 1995, 60, 788-789; Wang et al., Bioorganic & Medicinal Chemistry Letters, 1999, 9, 885-890; and Mikhailov et al., Nucleosides & Nucleotides, 1991, 10(1-3), 339-343) and Leonid et al., 1995, 14(3-5), 901-905). The synthesis of 2'-F modified nucleosides and their incorporation into oligomeric compounds is well known in published literature (see for example Issued U.S. Patents 6,005,087 and 5,670,633).

A genus of modified nucleosides including optional modifcation at a plurality of positions including the 5'-position and the 2'-position of the sugar ring and oligomeric compounds incorporating these modified nucleosides therein has been reported (see International Application Number: PCT/US94/02993, Published on October 13, 1994 as WO 94/22890).

There remains a long-felt need for agents that specifically regulate gene expression via antisense mechanisms. Disclosed herein are antisense compounds useful for modulating gene expression pathways, including those relying on mechanisms of action such as RNaseH, RNAi and dsRNA enzymes, as well as other antisense mechanisms based on target degradation or target occupancy. One having skill in the art, once armed with this disclosure will be able, without undue experimentation, to identify, prepare and exploit antisense compounds for these uses.

### BRIEF SUMMARY OF THE INVENTION

In certain aspects of the present invention compounds are provided having the formula: 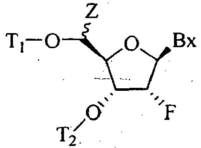 wherein:
Bx is an optionally modified heterocyclic base moiety;
T₁ is H or a hydroxyl protecting group;
T₂ is H, a hydroxyl protecting group or a reactive phosphorus group;
Z is C₁-C₆ alkyl, C₂-C₆ alkenyl, C₂-C₆ alkynyl, substituted C₁-C₆ alkyl, substituted C₂-C₆ alkenyl or substituted C₂-C₆ alkynyl, and
wherein each of the substituted groups, is, independently, mono or poly substituted with optional ly protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

In certain embodiments, Z is methyl, ethyl, vinyl, hydroxymethyl, aminomethylene, methoxymethylene, allyl, or propyl. In certain embodiments, Z is methyl.

In certain aspects of the present invention compounds are provided having the formula and specific configuration:

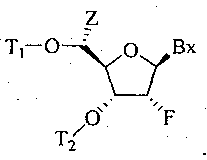

In certain aspects of the present invention compounds are provided having the formula and specific configuration:

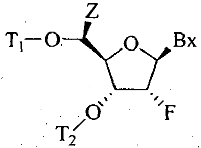

In certain embodiments, at least one of T₁ and T₂ is a hydroxyl protecting group. In certain embodiments, each of the hydroxyl protecting groups is, independently, selected from acetyl, benzyl, benzoyl, 2,6-dichlorobenzyl, t-butyldimethylsilyl, t-butyldiphenylsilyl, mesylate, tosylate, dimethoxytrityl (DMT), 9-phenylxanthine-9-yl (Pixyl) and 9-(p-methoxyphenyl)xanthine-9-yl (MOX). In certain embodiments, T₁ is a hydroxyl protecting group selected from acetyl, benzyl, t-butyldimethylsilyl, t-butyl-diphenylsilyl and dimethoxytrityl.

In certain embodiments, T₂ is a reactive phosphorus group. In certain embodiments, the reactive phosphorus group is diisopropylcyanoethoxy phosphoramidite or H-phosphonate. In certain embodiments, T₁ is 4,4'-dimethoxytrityl. In certain embodiments, T₁ is 4,4'-dimethoxytrityl and T₂ is diisopropylcyanoethoxy phosphoramidite.

In certain embodiments, Bx is a pyrimidine, modified pyrimidine, purine or a modified purine. In certain embodiments, Bx is uracil, 5-methyluracil, 5-thiazolo-uracil, 2-thio-uracil, 5-propynyl-uracil, thymine, 2'-thio-thymine, cytosine, 5-methylcytosine, 5-thiazolo-cytosine, 5-propynyl-cytosine, adenine, guanine or 2,6-diaminopurine In certain embodiments, Bx is uracil, 5-methyluracil, 5-propynyl-uracil, thymine, cytosine, 5-methylcytosine, 5-propynyl-cytosine, adenine or guanine.

In certain aspects of the present invention oligomeric compounds are provided comprising at least one monomer having formula I: 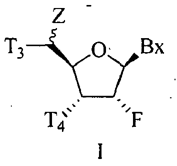 wherein independently for each of said at least one monomer of formula I:
Bx is an optionally modified heterocyclic base moiety;
T₃ is hydroxyl, a protected hydroxyl, a phosphate moiety, a linked conjugate group or an internucleoside linking group attaching said monomer of formula I to a nucleoside, a nucleotide, a monomeric subunit, an oligonucleoside, an oligonucleotide or an oligomeric compound;
each T₄ is, independently, is H, a hydroxyl protecting group, a linked conjugate group or an internucleoside linking group attaching said monomer of formula I to a nucleoside, a nucleotide, a monomeric subunit, an oligonucleoside, an oligonucleotide or an oligomeric compound;
wherein at least one of T₃ and T₄ is an internucleoside linking group attaching said monomer of formula I to a nucleoside, a nucleotide, a monomeric subunit, an oligonucleoside, an oligonucleotide, or an oligomeric compound;
Z is C₁-C₆ alkyl, C₂-C₆ alkenyl, C₂-C₆ alkynyl, substituted C₁-C₆ alkyl, substituted C₂-C₆ alkenyl or substituted C₂-C₆ alkynyl, and
wherein each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

In.certain embodiments, the oligomeric compounds comprise a plurality of monomers of formula I.

In certain embodiments, each Z is, independently, methyl, ethyl, vinyl, hydroxymethyl, aminomethyl, methoxymethyl, allyl, or propyl. In certain embodiments, at least one Z is methyl. In a preferred embodiment, each Z is methyl. In another preferred embodiment, each Z is methy and has the same stereochemistry.

In certain embodiments, each Bx is, independently selected from uracil, 5-methyluracil, 5-propynyl-uracil, thymine, cytosine, 5-methylcytosine, 5-propynyl-cytosine, adenine and guanine.

In certain aspects of the present invention oligomeric compounds are provided having at least one monomer of formula I having the specific configuration:

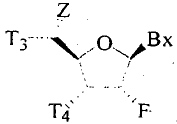

In certain aspects of the present invention oligomeric compounds are provided having a plurality of monomers of formula I, each having the specific configuration:

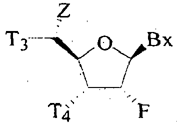

In certain aspects of the present invention oligomeric compounds are provided having at least one monomer of formula I having the specific configuration:

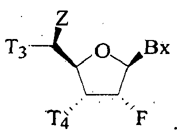

In certain aspects of the present invention oligomeric compounds are provided having a plurality of monomers of formula I, each having the specific configuration:

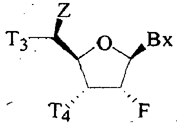

In certain embodiments, one T₃ is H or a hydroxyl protecting group. In certain embodiments, one T₃ is a phosphate group, substituted phosphate group, phosphorothioate group or a substituted phosphorothioate group. In certain embodiments, one T₄ is H or a hydroxyl protecting group.

In certain embodiments, oligomeric compounds are provided comprising at least one region of at least two contiguous monomers of formula I. In certain embodiments, oligomeric compounds are provided comprising at least two regions of at least two contiguous monomers of formula I. In certain embodiments, oligomeric compounds are provided comprising least two regions of at least two contiguous monomers of formula I wherein the oligomeric compounds comprise gapped oligomeric compounds. In certain embodiments, the gapped oligomeric compounds further comprise at least one region of from about 8 to about 14 contiguous β-D-2'-deoxyribofuranosyl nucleosides. In certain embodiments, the gapped oligomeric compounds further comprise at least one region of from about 8 to about 11 continuous P-D-2'-deoxyribofuranosyl nucleosides.

In certain embodiments, oligomeric compounds are provided comprising a first region of from 2 to 3 contiguous monomers, an optional third region having 1 monomer or 2 contiguous monomers, and a second region located between said first and said third regions comprising from 8 to 14 β-D-2'-deoxyribofuranosyl nucleosides wherein each of said monomers is a monomer of formula I. In certain embodiments, the second region comprises from 8 to 11β-D-2'-deoxyribofuranosyl nucleosides. In certain embodiments, the third region comprises 2 contiguous monomers of formula I. In certain embodiments, the third region comprises one monomer of formula I. In certain embodiments, the Z group for each of said monomers of formula I are in the R configuration. In certain embodiments, the Z group for each of said monomers of formula I are in the R configuration and each Z is methyl. In certain embodiments, the Z group for each of said monomers of formula I are in the S configuration. In certain embodiments, the Z group for each of said monomers of formula I are in the S configuration and each Z is methyl.

In certain embodiments, oligomeric compounds are provided having at least one monomer of formula I and comprising from about 8 to about 40 nucleosides and/or monomers in length. In certain embodiments, oligomeric compounds are provided having at least one monomer of formula I and comprising from about 8 to about 20 nucleosides and/or monomers in length. In certain embodiments, oligomeric compounds are provided having at least one monomer of formula I and comprising from about 10 to about 16 nucleosides and/or monomers in length. In certain embodiments, oligomeric compounds are provided having at least one monomer of formula I and comprising from about 10 to about 14 nucleosides and/or monomers in length.

In one aspect of the present invention methods of reducing target mRNA comprising contacting one or more cells, a tissue or an animal with an oligomeric compound comprising at least one monomer of formula I are provided.

In certain aspects of the present invention compositions are provided comprising first and second chemically synthesized oligomeric compounds, wherein the first oligomeric compound is fully complementary to the second oligomeric compound; the first oligomeric compound is fully complementary to a selected nucleic acid target; and at least one of said first and second oligomeric compounds comprises at least one monomer having formula I: 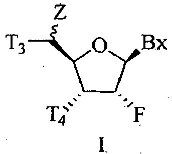 wherein independently for each of said at least one monomer of formula I:
Bx is an optionally modified heterocyclic base moiety;
T₃ is hydroxyl, a protected hydroxyl, a phosphate moiety, a linked conjugate group or an internucleoside linking group attaching said monomer of formula I to a nucleoside, a nucleotide, a monomeric subunit, an oligonucleoside, an oligonucleotide or an oligomeric compound;
each T₄ is, independently, is H, a hydroxyl protecting group, a linked conjugate group or an internucleoside linking group attaching said monomer of formula I to a nucleoside, a nucleotide, a monomeric subunit, an oligonucleoside, an oligonucleotide or an oligomeric compound;
wherein at least one of T₃ and T₄ is an internucleoside linking group attaching said monomer of formula I to a nucleoside, a nucleotide, a monomeric subunit, an oligonucleoside, an oligonucleotide, or an oligomeric compound;
Z is C₁-C₆ alkyl, C₂-C₆ alkenyl, C₂-C₆ alkynyl, substituted C₁-C₆ alkyl, substituted C₂-C₆ alkenyl or substituted C₂-C₆ alkynyl, and
wherein each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

In certain embodiments, the composition comprises a plurality of monomers of formula I.

In certain embodiments, the composition each Z is, independently, methyl, ethyl, vinyl, hydroxymethyl, aminomethyl, methoxymethyl, allyl, or propyl. In certain embodiments, at least one Z is methyl. In certain embodiments, each Z is methyl.

In certain embodiments, each Bx is, independently selected from uracil, 5-methyluracil, 5-propynyl-uracil, thymine, cytosine, 5-methylcytosine, 5-propynyl-cytosine, adenine and guanine.

In certain aspects of the present invention compositions are provided having at least one monomer of formula I having the specific configuration:

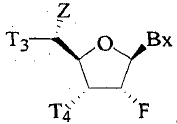

In certain aspects of the present invention compositions are provided having a plurality of monomers of formula I, each having the specific configuration:

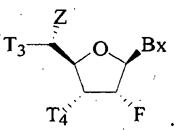

In certain aspects of the present invention compositions are provided having at least one monomer of formula I having the specific configuration:

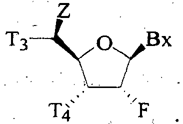

In certain aspects of the present invention compositions are provided having a plurality of monomers of formula I, each having the specific configuration:

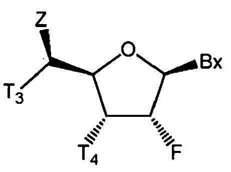

In certain embodiments, one T₃ is H or a hydroxyl protecting group.

In certain embodiments, the first strand of the composition is an antisense strand and the second strand is a sense strand.

In certain embodiments, compositions are provided wherein at least one strand comprises an alternating motif, a positional motif, a hemimer motif or a gapped motif. In certain embodiments, the strand comprising an alternating motif, a positional motif or a gapped motif is the antisense strand. In certain embodiments, the strand comprising an alternating motif, a positional motif or a gapped motif is the sense strand.

In certain embodiments, compositions are proved that further compre a phosphate moiety.

In certain embodiments, compositions are proved wherein each of said first and second oligomeric compounds comprises from about 17 to about 26 nucleosides and/or monomers in length. In certain embodiments, compositions are proved wherein each of said first and second oligomeric compounds comprises from about 19 to about 23 nucleosides and/or monomers in length. In certain embodiments, compositions are proved wherein each of said first and second oligomeric compounds comprises from about 19 to about 21 nucleosides and/or monomers in length.

One aspect of the present disclosure includes methods of reducing target mRNA comprising contacting one or more cells, a tissue or an animal with a composition comprising at least one monomer of formula I are provided.

### DETAILED DESCRIPTION OF THE INVENTION

The present invention provides 5'-substituted-2'-F modified nucleosides, oligomeric compounds that include such modified nucleosides and disclosed herein are methods of using the oligomeric compounds. Also included are intermediates and methods for preparing the 5'-substituted-2'-F modified nucleosides and the oliogmeric compounds. More particularly, the present invention provides 5'-substituted-2'-F modified nucleosides wherein a preferred 5'-substituent is a methyl group. In a preferred embodiment, the 5'-substituent group is in a particular configuration providing either the (R) or (S) isomer. In some embodiments, the oligomeric compounds and compositions of the present invention are designed to hybridize to a portion of a target RNA. In another embodiment, the oligomeric compounds of the present invention can be used in the design of aptamers which are oligomeric compounds capable of binding to aberrant proteins in an in vivo setting.

In certain aspects of the present invention, 5'-substituted-2'-F modified nucleosides are provided having the formula: 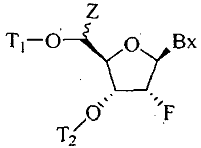 wherein:
Bx is an optionally modified heterocyclic base moiety;
T₁ is H or a hydroxyl protecting group;
T₂ is H, a hydroxyl protecting group or a reactive phosphorus group;
Z is C₁-C₆ alkyl, C₂-C₆ alkenyl, C₂-C₆ alkynyl, substituted C₁-C₆ alkyl, substituted C₂-C₆ alkenyl or substituted C₂-C₆ alkynyl, and
wherein each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

In certain aspects of the present invention, 5'-substituted-2'-F modified nucleosides are prepared having orthogonally protected reactive groups including a reactive phosphorus group. Such 5'-substituted-2'-F modified nucleosides are useful as monomers for oligomer synthesis. One illustrative example, which is not meant to be limiting, of such a 5'-substituted-2'-F modified nucleoside has the formula: 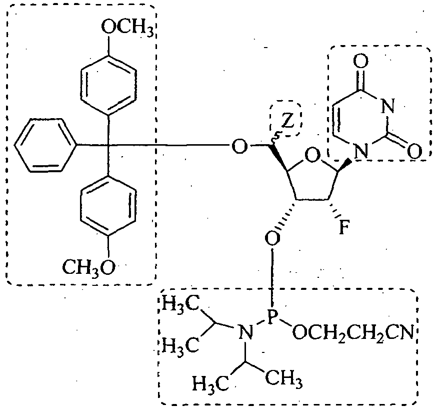 wherein the groups surrounded by broken lined boxes are variable. The Z group at the 5'position can be prepared in the R or S configuration. The 5'-substituted-2'-F modified nucleoside shown above is generically referred to as a dimethoxytrityl phosphoramidite.

In certain aspects of the present invention, oligomeric compounds are provided having at least one monomer of formula I: 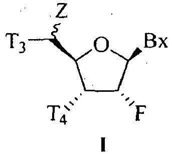 wherein independently for each of the at least one monomer of formula I:
Bx is an optionally modified heterocyclic base moiety;
T₃ is hydroxyl, a protected hydroxyl, a phosphate moiety, a linked conjugate group or an internucleoside linking group attaching said monomer of formula I to a nucleoside, a nucleotide, a monomeric subunit, an oligonucleoside, an oligonucleotide or an oligomeric compound;
each T₄ is, independently, is H, a hydroxyl protecting group, a linked conjugate group or an internucleoside linking group attaching said monomer of formula I to a nucleoside, a nucleotide, a monomeric subunit, an oligonucleoside, an oligonucleotide or an oligomeric compound;
wherein at least one of T₃ and T₄ is an internucleoside linking group attaching said monomer of formula I to a nucleoside, a nucleotide, a monomeric subunit, an oligonucleoside, an oligonucleotide, or an oligomeric compound;
Z is C₁-C₆ alkyl, C₂-C₆ alkenyl, C₂-C₆ alkynyl, substituted C₁-C₆ alkyl, substituted C₂-C₆ alkenyl or substituted C₂-C₆ alkynyl, and
wherein each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

In certain aspects of the invention, each monomer within an oligomeric compound the Z substituent can be in either the R or S configuration. In a preferred embodiment, the Z substituent of each monomer within an oligomeric compound has either R or S configuration.

The oligomeric compounds of the present invention may also include an optional phosphate moiety that can be located at either the 5' or the 3'-terminus but is preferred at the 5'-terminus. The phosphate moiety has the formula: 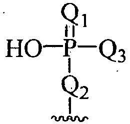 wherein
each of Q₁ and Q₂ is, independently, O or S; and Q₃ is OH or CH₃.

The 5'-substituted-2'-F modified nucleosides of the present invention are useful for modifying oligomeric compounds at one or more positions to enhance desired properties of oligomeric compounds in which they are incorporated such as nuclease resistance. Oligomeric compounds comprising such modified nucleosides are also expected to be useful as primers and probes in various diagnostic applications.

The 5'-substituted-2'-F modified nucleosides of the present invention are useful for modifying oligomeric compounds at one or more positions. Such modified oligomeric compounds can be described as having a particular motif. Motifs amenable to the present invention include but are not limited to a gapped motif, a hemimer motif, a blockmer motif, a fully modified motif, a positionally modified motif and an alternating motif. In conjunction with these motifs a wide variety of linkages can also be used including but not limited to phosphodiester and phosphorothioate linkages used uniformly or in combinations. The positioning of 5'-substituted-2'-F modified nucleosides and the use of linkage strategies can be optimized to enhance activity for a selected target. Such motifs can be further modified by the inclusion of 5'-and/or 3'-terminal groups including but not limited to further modified or unmodified nucleosides, conjugate groups and phosphate moieties.

The term "motif" refers to the distribution of sugar modified nucleosides within an oligomeric compound. The pattern is defined by the positioning of one type of sugar modified nucleosides relative to the positioning of other sugar modified nucleosides and/or unmodified nucleosides (β-D-ribonucleosides and/or β-D-deoxyribonucleosides). More specifically, the motif of a particular oligomeric compound is determined by the positioning of different sugar groups relative to each other. The type of heterocyclic base and internucleoside linkages used at each position is variable and is not a factor in determining the motif of an oligomeric compound. The presence of one or more other groups including terminal groups, protecting groups or capping groups is also not a factor in determining the motif.

A number of publications disclose motifs including hemimer motifs, blockmer motifs, gapped motifs, fully modified motifs, positionally modified motifs and alternating motifs, that can be used in a single stranded oligomeric compound or in one or both strands of a double strand duplex comprising two oligomeric compounds. Representative U.S. patents that teach the preparation of representative motifs include, but are not limited to, 5,013,830; 5,149,797; 5,220,007; 5,256,775; 5,366,878; 5,403,711; 5,491,133; 5,565,350; 5,623,065; 5,652,355; 5,652,356; and 5,700,922, certain of which are commonly owned with the instant application. Motifs are also disclosed in published International Applications PCT/US2005/019219, filed June 2, 2005 and published as WO 2005/121371 on December 22, 2005; PCT/US2005/019220, filed June 2, 2005, published as WO 2005/121372 on December 22, 2005; WO 2004/044133 published May 27, 2004, 3'-endo motifs; WO 2004/113496 published December 29, 2004, 3'-endo motifs; WO 2004/044136 published May 27, 2004, alternating motifs; WO 2004/044140 published May 27, 2004, 2'-modified motifs; WO 2004/043977 published May 27, 2004, 2'-F motifs; WO 2004/043978 published May 27, 2004, 2'-OCH₃ motifs; WO 2004/041889 published May 21, 2004, polycyclic sugar motifs; WO 2004/043979 published May 27, 2004, sugar surrogate motifs; and WO 2004/044138 published May 27, 2004, chimeric motifs; also see published US Application US20050080246 published April 14, 2005.

As used in the present invention the term "gapmer" or "gapped oligomeric compound" is meant to include a contiguous sequence of nucleosides divided into 3 regions, an internal region having an external region on each of the 5' and 3' ends. The regions are differentiated from each other at least by having different sugar groups that comprise the nucleosides. The types of nucleosides that are generally used to differentiate the regions of a gapped oligomeric compound include β-D-ribonucleosides, β-D-deoxyribonucleosides, 2'-modified nucleosides, 4'-thio modified nucleosides, 4'-thio-2'-modified nucleosides, and bicyclic sugar modified nucleosides. Each of the regions of a gapped oligomeric compound is essentially uniformly modified e.g. the sugar groups are identical with at least the internal region having different sugar groups than each of the external regions. The internal region or the gap generally comprises β-D-deoxyribo-nucleosides but can be a sequence of sugar modified nucleosides. A preferred gapped oligomeric compound according to the present invention comprises an internal region of β-D-deoxyribonucleosides with both of the external regions comprising monomers of formula I. Examples of gapped oliomeric compounds are illustrated in examples 17-22.

In a preferred embodiment, gapped oligomeric compounds are provided comprising one or two monomers of formula I at the 5'-end, two or three monomers of formula I at the 3'-end and an internal region of from 10 to 16 nucleosides. In another preferred embodiment, gapped oligomeric compounds are provided comprising one monomer of formula I at the 5'-end, two monomers of formula I at the 3'-end and an internal region of from 10 to 16 nucleosides. In a further preferred embodiment, gapped oligomeric compounds are provided comprising one monomer of formula I at the 5'-end, two monomers of formula I at the 3'-end and an internal region of from 10 to 14 nucleosides. In another preferred embodiment the internal region is essentially a contiguous sequence of β-D-deoxyribonucleosides. In another embodiment, oligomeric compounds are provided that further include one or more 5'- and/or 3'-terminal groups including but not limited to further modified or unmodified nucleosides, conjugate groups, phosphate moieties and other useful groups known to the art skilled. In a further preferred embodiment, each of the monomers of formula I have the configuration:

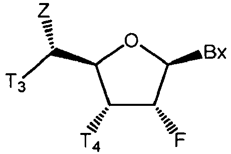

In another preferred embodiment, each of the monomers of formula I have the configuration:

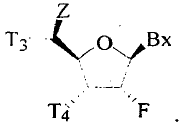

In certain embodiments, gapped oligomeric compounds are provided comprising from about 8 to about 40 nucleosides and/or monomers in length. In certain embodiments, gapped oligomeric compounds are provided comprising a mixture of from about 8 to about 20 nucleosides and monomers in length. In certain embodiments, gapped oligomeric compounds are provided comprising a mixture of from about 12 to about 23 nucleosides and monomers in length. In certain embodiments, gapped oligomeric compounds are provided comprising a mixture of from about 12 to about 16 nucleosides and monomers in length. In certain embodiments, gapped oligomeric compounds are provided comprising a mixture of from about 10 to about 16 nucleosides and monomers in length. In certain embodiments, gapped oligomeric compounds are provided comprising a mixture of from about 12 to about 14 nucleosides and monomers in length. In certain embodiments, gapped oligomeric compounds are provided comprising a mixture of from about 10 to about 14 nucleosides and monomers in length.

The terms "substituent" and "substituent group," as used herein, are meant to include groups that are typically added to other groups or parent compounds to enhance desired properties or give desired effects. Substituent groups can be protected or unprotected and can be added to one available site or to many available sites in a parent compound. Substituent groups may also be further substituted with other substituent groups and may be attached directly or via a linking group such as an alkyl or hydrocarbyl group to a parent compound. Such groups include without limitation, halogen, hydroxyl, alkyl, alkenyl, alkynyl, acyl (-C(O)Rₐₐ), carboxyl (-C(O)O-Rₐₐ), aliphatic groups, alicyclic groups, alkoxy, substituted oxy (-O-Rₐₐ), aryl, aralkyl, heterocyclic, heteroaryl, heteroarylalkyl, amino (-NR_{bb}R_{cc}), imino(=NR_{bb}), amido (-C(O)N-R_{bb}R_{cc} or -N(R_{bb})C(O)Rₐₐ), azido (-N₃), nitro (-NO₂), cyano (-CN), carbamido (-OC(O)NR_{bb}R_{cc} or -N(R_{bb})C(O)ORₐₐ), ureido (-N(R_{bb})C(O)NR_{bb}R_{cc}), thioureido (-N(R_{bb})C(S)NR_{bb}R_{cc}), guanidinyl (-N(R_{bb})-C(=NR_{bb})NR_{bb}R_{cc}), amidinyl (-C(=NR_{bb})NR_{bb}R_{cc} or -N(Rb_{b})C(NRb_{b})Rₐₐ), thiol (-SR_{bb}), sulfinyl (-S(O)R_{bb}), sulfonyl (-S(O)₂R_{bb}), sulfonamidyl (-S(O)₂NR_{bb}R_{cc} or -N(R_{bb})S(O)₂R_{bb}) and conjugate groups. Wherein each Rₐₐ, R_{bb} and R_{cc} is, independently, H, an optionally linked chemical functional group or a further substituent group with a preferred list including, without limitation H, alkyl, alkenyl, alkynyl, aliphatic, alkoxy, acyl, aryl, aralkyl, heteroaryl, alicyclic, heterocyclic and heteroarylalkyl. Selected substituents within the compounds described herein are present to a recursive degree.

In this context, "recursive substituent" means that a substituent may recite another instance of itself. Because of the recursive nature of such substituents, theoretically, a large number may be present in any given claim. One of ordinary skill in the art of medicinal chemistry and organic chemistry understands that the total number of such substituents is reasonably limited by the desired properties of the compound intended. Such properties include, by way of example and not limitation, physical properties such as molecular weight, solubility or log P, application properties such as activity against the intended target, and practical properties such as ease of synthesis.

Recursive substituents are an intended aspect of the invention. One of ordinary skill in the art of medicinal and organic chemistry understands the versatility of such substituents. To the degree that recursive substituents are present in a claim of the invention, the total number will be determined as set forth above.

The term "alkyl," as used herein, refers to a saturated straight or branched hydrocarbon radical containing up to twenty four carbon atoms. Examples of alkyl groups include, but are not limited to, methyl, ethyl, propyl, butyl, isopropyl, n-hexyl, octyl, decyl, dodecyl and the like. Alkyl groups typically include from 1 to about 24 carbon atoms, more typically from 1 to about 12 carbon atoms (C₁-C₁₂ alkyl) with from 1 to about 6 carbon atoms being more preferred. The term "lower alkyl" as used herein includes from 1 to about 6 carbon atoms.

The term "alkenyl," as used herein, refers to a straight or branched hydrocarbon chain radical containing up to twenty four carbon atoms and having at least one carbon-carbon double bond. Examples of alkenyl groups include, but are not limited to, ethenyl, propenyl, butenyl, I-methyl-2-buten-1-yl, dienes such as 1,3-butadiene and the like. Alkenyl groups typically include from 2 to about 24 carbon atoms, more typically from 2 to about 12 carbon atoms with from 2 to about 6 carbon atoms being more preferred.

The term "alkynyl," as used herein, refers to a straight or branched hydrocarbon radical containing up to twenty four carbon atoms and having at least one carbon-carbon triple bond. Examples of alkynyl groups include, but are not limited to, ethynyl, 1-propynyl, 1-butynyl, and the like. Alkynyl groups typically include from 2 to about 24 carbon atoms, more typically from 2 to about 12 carbon atoms with from 2 to about 6 carbon atoms being more preferred.

The term "acyl," as used herein, refers to a radical formed by removal of a hydroxyl group from an organic acid and has the general formula -C(O)-X where X is typically aliphatic, alicyclic or aromatic. Examples include aliphatic carbonyls, aromatic carbonyls, aliphatic sulfonyls, aromatic sulfinyls, aliphatic sulfinyls, aromatic phosphates, aliphatic phosphates and the like. Acyl groups as used herein may optionally include further substituent groups.

The term "alicyclic" or "alicyclyl" refers to a cyclic ring system wherein the ring is aliphatic. The ring system can comprise one or more rings wherein at least one ring is aliphatic. Preferred alicyclics include rings having from about 5 to about 9 carbon atoms in the ring. Alicyclic as used herein may optionally include further substituent groups.

The term "aliphatic," as used herein, refers to a straight or branched hydrocarbon radical containing up to twenty four carbon atoms wherein the saturation between any two carbon atoms is a single, double or triple bond. An aliphatic group preferably contains from 1 to about 24 carbon atoms, more typically from 1 to about 12 carbon atoms with from 1 to about 6 carbon atoms being more preferred. The straight or branched chain of an aliphatic group may be interupted with one or more heteroatoms that include nitrogen, oxygen, sulfur and phosphorus. Such aliphatic groups interupted by heteroatoms include without limitation polyalkoxys, such as polyalkylene glycols, polyamines, and polyimines. Aliphatic groups as used herein may optionally include further substitutent groups.

The term "alkoxy," as used herein, refers to a radical formed between an alkyl group and an oxygen atom wherein the oxygen atom is used to attach the alkoxy group to a parent molecule. Examples of alkoxy groups include, but are not limited to, methoxy, ethoxy, propoxy, isopropoxy, *n*-butoxy, sec-butoxy, *tert-*butoxy, n-pentoxy, neopentoxy, n-hexoxy and the like. Alkoxy groups as used herein may optionally include further substitutent groups.

The term "aminoalkyl" as used herein, refers to an amino substituted alkyl radical. This term is meant to include C₁-C₁₂ alkyl groups having an amino substituent at any position and wherein the alkyl group attaches the aminoalkyl group to the parent molecule. The alkyl and/or amino portions of the aminoalkyl group can be further substituted with substituent groups.

The terms "aralkyl" and "arylalkyl," as used herein, refer to a radical formed between an alkyl group and an aryl group wherein the alkyl group is used to attach the aralkyl group to a parent molecule. Examples include, but are not limited to, benzyl, phenethyl and the like. Aralkyl groups as used herein may optionally include further substitutent groups attached to the alkyl, the aryl or both groups that form the radical group.

The terms "aryl" and "aromatic," as used herein, refer to a mono- or polycyclic carbocyclic ring system radicals having one or more aromatic rings. Examples of aryl groups include, but are not limited to, phenyl, naphthyl, tetrahydronaphthyl, indanyl, idenyl and the like. Preferred aryl ring systems have from about 5 to about 20 carbon atoms in one or more rings. Aryl groups as used herein may optionally include further substitutent groups.

The terms "halo" and "halogen," as used herein, refer to an atom selected from fluorine, chlorine, bromine and iodine.

The terms "heteroaryl," and "heteroaromatic," as used herein, refer to a radical comprising a mono-or poly-cyclic aromatic ring, ring system or fused ring system wherein at least one of the rings is aromatic and includes one or more heteroatom. Heteroaryl is also meant to include fused ring systems including systems where one or more of the fused rings contain no heteroatoms. Heteroaryl groups typically include one ring atom selected from sulfur, nitrogen or oxygen. Examples of heteroaryl groups include, but are not limited to, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, benzimidazolyl, benzooxazolyl, quinoxalinyl, and the like. Heteroaryl radicals can be attached to a parent molecule directly or through a linking moiety such as an aliphatic group or hetero atom. Heteroaryl groups as used herein may optionally include further substitutent groups.

The term "heteroarylalkyl," as used herein, refers to a heteroaryl group as previously defined having an alky radical that can attach the heteroarylalkyl group to a parent molecule. Examples include, but are not limited to, pyridinylmethyl, pyrimidinylethyl, napthyridinylpropyl and the like. Heteroarylalkyl groups as used herein may optionally include further substitutent groups on one or both of the heteroaryl or alkyl portions.

The term "heterocyclic radical" as used herein, refers to a radical mono-, or poly-cyclic ring system that includes at least one heteroatom and is unsaturated, partially saturated or fully saturated, thereby including heteroaryl groups. Heterocyclic is also meant to include fused ring systems wherein one or more of the fused rings contain at least one heteroatom and the other rings can contain one or more heteroatoms or optionally contain no heteroatoms. A heterocyclic group typically includes at least one atom selected from sulfur, nitrogen or oxygen. Examples of heterocyclic groups include, [1,3]dioxolane, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, quinoxalinyl, pyridazinonyl, tetrahydrofuryl and the like. Heterocyclic groups as used herein may optionally include further substitutent groups.

The term "hydrocarbyl" includes groups comprising C, O and H. Included are straight, branched and cyclic groups having any degree of saturation. Such hydrocarbyl groups can include one or more heteroatoms selected from N, O and S and can be further mono or poly substituted with one or more substituent groups.

The term "mono or poly cyclic structure" as used in the present invention includes all ring systems that are single or polycyclic having rings that are fused or linked and is meant to be inclusive of single and mixed ring systems individually selected from aliphatic, alicyclic, aryl, heteroaryl, aralkyl, arylalkyl, heterocyclic, heteroaryl, heteroaromatic, heteroarylalkyl. Such mono and poly cyclic structures can contain rings that are uniform or have varying degrees of saturation including fully saturated, partially saturated or fully unsaturated. Each ring can comprise ring atoms selected from C, N, O and S to give rise to heterocyclic rings as well as rings comprising only C ring atoms which can be present in a mixed motif such as for example benzimidazole wherein one ring has only carbon ring atoms and the fused ring has two nitrogen atoms. The mono or poly cyclic structures can be further substituted with substituent groups such as for example phthalimide which has two =O groups attached to one of the rings. In another aspect, mono or poly cyclic structures can be attached to a parent molecule directly through a ring atom, through a substituent group or a bifunctional linking moiety.

The term "oxo" refers to the group (=O).

The terms "bicyclic nucleic acid (BNA)" and "bicyclic nucleoside" as used in the present invention includes nucleosides wherein the furanose ring includes a bridge connecting two of the ring's non-geminal carbon atoms, thereby forming a bicyclic ring system.

Linking groups or bifunctional linking moieties such as those known in the art are amenable to the present invention. Linking groups are useful for attachment of chemical functional groups, conjugate groups, reporter groups and other groups to selective sites in a parent compound such as for example an oligomeric compound. In general a bifunctional linking moiety comprises a hydrocarbyl moiety having two functional groups. One of the functional groups is selected to bind to a parent molecule or compound of interest and the other is selected to bind essentially any selected group such as a chemical functional group or a conjugate group. In some embodiments, the linker comprises a chain structure or an oligomer of repeating units such as ethylene glyol or amino acid units. Examples of functional groups that are routinely used in bifunctional linking moieties include, but are not limited to, electrophiles for reacting with nucleophilic groups and nucleophiles for reacting with electrophilic groups. In some embodiments, bifunctional linking moieties include amino, hydroxyl, carboxylic acid, thiol, unsaturations (e.g., double or triple bonds), and the like. Some nonlimiting examples of bifunctional linking moieties include 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other linking groups include, but are not limited to, substituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

In one aspect of the present invention oligomeric compounds are modified by covalent attachment of one or more conjugate groups. In general, conjugate groups modify one or more properties of the attached oligomeric compound including but not limited to pharmakodynamic, pharmacokinetic, binding, absorption, cellular distribution, cellular uptake, charge and clearance. Conjugate groups are routinely used in the chemical arts and are linked directly or via an optional linking moiety or linking group to a parent compound such as an oligomeric compound. A preferred list of conjugate groups includes without limitation, intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins and dyes.

In another aspect of the present invention, oligomeric compounds are modified by covalent attachemnt of one or more 5' or 3'-terminal groups that include but are not limited to further modified or unmodified nucleosides, conjugate groups and phosphate moieties. Such terminal groups can be useful for enhancing properties of oligomeric compounds such as for example nuclease stability, uptake and delivery.

The term "protecting group," as used herein, refers to a labile chemical moiety which is known in the art to protect reactive groups including without limitation, hydroxyl, amino and thiol groups, against undesired reactions during synthetic procedures. Protecting groups are typically used selectively and/or orthogonally to protect sites during reactions at other reactive sites and can then be removed to leave the unprotected group as is or available for further reactions. Protecting groups as known in the art are described generally in Greene and Wuts, Protective Groups in Organic Synthesis, 3rd edition, John Wiley & Sons, New York (1999).

Groups can be selectively incorporated into oligomeric compounds of the invention as precursors. For example an amino group can be placed into a compound of the invention as an azido group that can be chemically converted to the amino group at a desired point in the synthesis. Generally, groups are protected or present as precursors that will be inert to reactions that modify other areas of the parent molecule for conversion into their final groups at an appropriate time. Further representative protecting or precursor groups are discussed in Agrawal, et al., Protocols for Oligonucleotide Conjugates, Eds, Humana Press; New Jersey, 1994; Vol. 26 pp. 1-72.

Examples of hydroxyl protecting groups include, but are not limited to, acetyl, t-butyl, t-butoxymethyl, methoxymethyl, tetrahydropyranyl, 1-ethoxyethyl, 1-(2-chloroethoxy)ethyl, p-chlorophenyl, 2,4-dinitrophenyl, benzyl, 2,6-dichlorobenzyl, diphenylmethyl, p-nitrobenzyl, bis(2-acetoxyethoxy)methyl (ACE), 2-trimethylsilylethyl, trimethylsilyl, triethylsilyl, t-butyldimethylsilyl, t-butyldiphenylsilyl, triphenylsilyl, [(triisopropylsilyl)oxy]methyl (TOM), benzoylformate, chloroacetyl, trichloroacetyl, trifluoroacetyl, pivaloyl, benzoyl, p-phenylbenzoyl, 9-fluorenylmethyl carbonate, mesylate, tosylate, triphenylmethyl (trityl), monomethoxytrityl, dimethoxytrityl (DMT), trimethoxytrityl, 1(2-fluorophenyl)-4-methoxypiperidin-4-yl (FPMP), 9-phenylxanthine-9-yl (Pixyl) and 9-(p-methoxyphenyl)xanthine-9-yl (MOX). Where more preferred hydroxyl protecting groups include, but are not limited to, benzyl, 2,6-dichlorobenzyl, t-butyldimethylsilyl, t-butyldiphenylsilyl, benzoyl, mesylate, tosylate, dimethoxytrityl (DMT), 9-phenylxanthine-9-yl (Pixyl) and 9-(p-methoxyphenyl)xanthine-9-yl (MOX).

Examples of amino protecting groups include, but are not limited to, carbamate-protecting groups, such as 2-trimethylsilylethoxycarbonyl (Teoc), 1-methyl-1-(4-biphenylyl)ethoxycarbonyl (Bpoc), t-butoxycarbonyl (BOC), allyloxycarbonyl (Alloc), 9-fluorenylmethyloxycarbonyl (Fmoc), and benzyloxycarbonyl (Cbz); amide-protecting groups, such as formyl, acetyl, trihaloacetyl, benzoyl, and nitrophenylacetyl; sulfonamide-protecting groups, such as 2-nitrobenzenesulfonyl; and imine- and cyclic imide-protecting groups, such as phthalimido and dithiasuccinoyl.

Examples of thiol protecting groups include, but are not limited to, triphenylmethyl (trityl), benzyl (Bn), and the like.

In some preferred embodiments oligomeric compounds are prepared by connecting nucleosides with optionally protected phosphorus containing internucleoside linkages. Representative protecting groups for phosphorus containing internucleoside linkages such as phosphodiester and phosphorothioate linkages include β-cyanoethyl, diphenylsilylethyl, δ-cyanobutenyl, cyano p-xylyl (CPX), N-methyl-N-trifluoroacetyl ethyl (META), acetoxy phenoxy ethyl (APE) and butene-4-yl groups. See for example U.S. Patents Nos. 4,725,677 and Re. 34,069 (β-cyanoethyl); Beaucage, S.L. and lyer, R.P., Tetrahedron, 49 No. 10, pp. 1925-1963 (1993); Beaucage, S.L. and lyer, R.P., Tetrahedron, 49 No. 46, pp. 10441-10488 (1993); Beaucage, S.L. and Iyer, R.P., Tetrahedron, 48 No. 12, pp. 2223-2311 (1992).

The term "orthogonally protected" refers to functional groups which are protected with different classes of protecting groups, wherein each class of protecting group can be removed in any order and in the presence of all other classes (see, Barany, G. and Merrifield, R.B., J. Am. Chem. Soc., 1977, 99, 7363; *idem,* 1980, 102, 3084.) Orthogonal protection is widely used in for example automated oligonucleotide synthesis. A functional group is deblocked in the presence of one or more other protected functional groups which is not affected by the deblocking procedure. This deblocked functional group is reacted in some manner and at some point a further orthogonal protecting group is removed under a different set of reaction conditions. This allows for selective chemistry to arrive at a desired compound or oligomeric compound.

The present invention provides compounds having reactive phosphorus groups useful for forming internucleoside linkages including for example phosphodiester and phosphorothioate internucleoside linkages. Such reactive phosphorus groups are known in the art and contain phosphorus atoms in P^{III} or P^{V} valence state including, but not limited to, phosphoramidite, H-phosphonate, phosphate triesters and phosphorus containing chiral auxiliaries. A preferred synthetic solid phase synthesis utilizes phosphoramidites (P^{III} chemistry) as reactive phosphites. The intermediate phosphite compounds are subsequently oxidized to the P^{V} state using known methods to yield, in preferred embodiments, phosphodiester or phosphorothioate internucleotide linkages. Additional reactive phosphates and phosphites are disclosed in Tetrahedron Report Number 309 (Beaucage and Iyer, Tetrahedron, 1992, 48, 2223-2311).

Specific examples of oligomeric compounds useful in this invention include oligonucleotides containing modified e.g. non-naturally occurring internucleoside linkages. Two main classes of internucleoside linkages are defined by the presense or absence of a phosphorus atom. Modified internucleoside linkages having a phosphorus atom include, but are not limited to, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and boranophosphates having normal 3'-5' linkages, 2'-5' linked analogs of these, and those having inverted polarity wherein one or more internucleotide linkages is a 3' to 3', 5' to 5' or 2' to 2' linkage. Oligonucleotides having inverted polarity can comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included.

Representative U.S. patents that teach the preparation of the above phosphorus-containing linkages include, but are not limited to, U.S.: 3,687,808; 4,469,863; 4,476,301; 5,023,243; 5,177,196; 5,188,897; 5,264,423; 5,276,019; 5,278,302; 5,286,717; 5,321,131 ; 5,399,676; 5,405,939; 5,453,496; 5,455,233; 5,466,677; 5,476,925; 5,519,126; 5,536,821 ; 5,541,306; 5,550,111; 5,563,253; 5,571,799; 5,587,361; 5,194,599; 5,565,555; 5,527,899; 5,721,218; 5,672,697 and 5,625,050, certain of which are commonly owned with this application.

Modified internucleoside linkages not having a phosphorus atom include, but are not limited to, those that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH₂ component parts. In the context of this invention, the term "oligonucleoside" refers to a sequence of nucleosides that are joined by internucleoside linkages that do not have phosphorus atoms.

Representative U.S. patents that teach the preparation of the above oligonucleosides include, but are not limited to, U.S.: 5,034,506; 5,166,315; 5,185,444; 5,214,134; 5,216,141 ; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; 5,792,608; 5,646,269 and 5,677,439, certain of which are commonly owned with this application.

The compounds (e.g., 5'-substituted-2'-F modified nucleosides) described herein can be prepared by any of the applicable techniques of organic synthesis, as, for example, illustrated in the examples below. Many such techniques are well known in the art. However, many of the known techniques are elaborated in Compendium of Organic Synthetic Methods (John Wiley & Sons, New York) Vol. 1 , Ian T. Harrison and Shuyen Harrison (1971); Vol. 2, Ian T. Harrison and Shuyen Harrison (1974); Vol. 3, Louis S. Hegedus and Leroy Wade (1977); Vol. 4, Leroy G. Wade Jr., (1980); Vol. 5, Leroy G. Wade Jr. (1984); and Vol. 6, Michael B. Smith; as well as March, J., Advanced Organic Chemistry, 3rd Edition, John Wiley & Sons, New York (1985); Comprehensive Organic Synthesis. Selectivity, Strategy & Efficiency in Modern Organic Chemistry, In 9 Volumes, Barry M. Trost, Editor-in-Chief, Pergamon Press, New York (1993); Advanced Organic Chemistry, Part B: Reactions and Synthesis, 4th Ed.; Carey and Sundberg; Kluwer Academic/Plenum Publishers: New York (2001); Advanced Organic Chemistry, Reactions, Mechanisms , and Structure, 2nd Edition, March, McGraw Hill (1977); Protecting Groups in Organic Synthesis, 2nd Edition, Greene, T. W., and Wutz, P.G.M., John Wiley & Sons, New York (1991); and Comprehensive Organic Transformations, 2nd Edition, Larock, R.C., John Wiley & Sons, New York (1999).

The compounds described herein contain one or more asymmetric centers and thus give rise to enantiomers, diastereomers, and other stereoisomeric forms that may be defined, in terms of absolute stereochemistry, as (R)- or (S)-, a or β, or as (D)- or (L)- such as for amino acids. The present invention is meant to include all such possible isomers, as well as their racemic and optically pure forms. Optical isomers may be prepared from their respective optically active precursors by the procedures described above, or by resolving the racemic mixtures. The resolution can be carried out in the presence of a resolving agent, by chromatography or by repeated crystallization or by some combination of these techniques which are known to those skilled in the art. Further details regarding resolutions can be found in Jacques, et al., Enantiomers, Racemates, and Resolutions (John Wiley & Sons, 1981). When the compounds described herein contain olefinic double bonds, other unsaturation, or other centers of geometric asymmetry, and unless specified otherwise, it is intended that the compounds include both E and Z geometric isomers or cis- and trans-isomers. Likewise, all tautomeric forms are also intended to be included. The configuration of any carbon-carbon double bond appearing herein is selected for convenience only and is not intended to designate a particular configuration unless the text so states; thus a carbon-carbon double bond or carbon-heteroatom double bond depicted arbitrarily herein as trans may be cis, trans, or a mixture of the two in any proportion.

In the context of the present invention, the term "oligomeric compound" refers to a polymer having at least a region that is capable of hybridizing to a nucleic acid molecule. The term "oligomeric compound" includes oligonucleotides, oligonucleotide analogs and oligonucleosides as well as nucleotide mimetics and/or mixed polymers comprising nucleic acid and non-nucleic acid components and chimeric oligomeric compounds comprising mixtures of nucleosides from any of these catagories. Oligomeric compounds are routinely prepared linearly but can be joined or otherwise prepared to be circular and may also include branching. Oligomeric compounds can form double stranded constructs such as for example two strands hybridized to form double stranded compositions. The double stranded compositions can be linked or separate and can include overhangs on the ends. In general, an oligomeric compound comprises a backbone of linked monomeric subunits where each linked monomeric subunit is directly or indirectly attached to a heterocyclic base moiety. Oligomeric compounds may also include monomeric subunits that are not linked to a heterocyclic base moiety thereby providing abasic sites. The linkages joining the monomeric subunits, the sugar moieties or surrogates and the heterocyclic base moieties can be independently modified. The linkage-sugar unit, which may or may not include a heterocyclic base, may be substituted with a mimetic such as the monomers in peptide nucleic acids. The ability to modify or substitute portions or entire monomers at each position of an oligomeric compound gives rise to a large number of possible motifs.

As is known in the art, a nucleoside is a base-sugar combination. The base portion of the nucleoside is normally a heterocyclic base moiety. The two most common classes of such heterocyclic bases are purines and pyrimidines. Nucleotides are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. The respective ends of this linear polymeric structure can be joined to form a circular structure by hybridization or by formation of a covalent bond. However, open linear structures are generally desired. Within the oligonucleotide structure, the phosphate groups are commonly referred to as forming the internucleoside linkages of the oligonucleotide. The normal internucleoside linkage of RNA and DNA is a 3' to 5' phosphodiester linkage.

The term "nucleobase" or "heterocyclic base moiety" as used herein, is intended to by synonymous with "nucleic acid base or mimetic thereof." In general, a nucleobase or heterocyclic base moiety is any substructure that contains one or more atoms or groups of atoms capable of hydrogen bonding to a base of a nucleic acid.

In the context of this invention, the term "oligonucleotide" refers to an oligomer or polymer of ribonucleic acid (RNA) or deoxyribonucleic acid (DNA). This term includes oligonucleotides composed of naturally-occurring nucleobases, sugars and covalent internucleoside linkages. The term "oligonucleotide analog" refers to oligonucleotides that have one or more non-naturally occurring portions. Such non-naturally occurring oligonucleotides are often desired over naturally occurring forms because of desirable properties such as, for example, enhanced cellular uptake, enhanced affinity for nucleic acid target and increased stability in the presence of nucleases.

As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine, 3-deazaguanine and 3-deazaadenine, universal bases, hydrophobic bases, promiscuous bases, size-expanded bases, and fluorinated bases as defined herein. Further modified nucleobases include tricyclic pyrimidines such as phenoxazine cytidine(1H-pyrimido[5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in United States Patent No. 3,687,808, those disclosed in The Concise Encyclopedia Of Polymer Science And Engineering, pages 858-859, Kroschwitz, J.I., ed. John Wiley & Sons, 1990**,** those disclosed by Englisch et al., Angewandte Chemie, International Edition, 1991, 30, 613, and those disclosed by Sanghvi, Y.S., Chapter 15, Antisense Research and Applications, pages 289-302, Crooke, S.T. and Lebleu, B., ed., CRC Press, 1993**.**

Modified nucleobases include, but are not limited to, universal bases, hydrophobic bases, promiscuous bases, size-expanded bases, and fluorinated bases as defined herein. Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2 °C (Sanghvi, Y.S., Crooke, S.T. and Lebleu, B., eds., Antisense Research and Applications, CRC Press, Boca Raton, 1993, pp. 276-278) and are presently preferred base substitutions, even more particularly when combined with 2'-O-methoxyethyl sugar modifications.

Representative United States patents that teach the preparation of certain of the above noted modified nucleobases as well as other modified nucleobases include, but are not limited to, the above noted U.S. 3,687,808, as well as U.S.: 4,845,205; 5,130,302; 5,134,066; 5,175,273; 5,367,066; 5,432,272; 5,457,187; 5,459,255; 5,484,908; 5,502,177; 5,525,711 ; 5,552,540; 5,587,469; 5,594,121 ; 5,596,091; 5,614,617; 5,645,985; 5,750,692; 5,830,653; 5,763,588; 6,005,096; and 5,681,941, certain of which are commonly owned with the instant application.

Oligomeric compounds of the present invention may also contain one or more nucleosides having modified sugar moieties. The furanosyl sugar ring can be modified in a number of ways including substitution with a substituent group (2', 3', 4' or 5'), bridging to form a BNA, substitution of the 4'-0 with a heteroatom such as S or N(R) or some combination of these such as a 4'-S-2'-substituted nucleoside. Some representative U.S. patents that teach the preparation of such modified sugars include, but are not limited to, U.S.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; 5,700,920; 6,600,032 and International Application PCT/US2005/019219, filed June 2, 2005 and published as WO 2005/121371 on December 22, 2005 certain of which are commonly owned with the instant application. A representative list of preferred modified sugars includes but is not limited to substituted sugars having a 2'-F, 2'-OCH₂ or a 2'-O(CH₂)₂-OCH₃ (2'-MOE or simply MOE) substituent group; 4'-thio modified sugars and bicyclic modified sugars.

As used herein the terms "sugar surrogate", "mimetic" and "sugar mimetic" are meant to include nucleosides wherein the sugar group has been substituted with a non-furanose type group such as for example a morpholino or bicyclo[3.1.0]hexyl furanose replacement group. The linkage group can also be modified in conjunction with the sugar surrogate or sugar mimetic such as for example peptide nucleic acids or morpholinos (morpholinos linked by -N(H)-C(=O)-O- or other non-phosphodiester linkage).

The oligomeric compounds in accordance with the present invention can comprise from about 8 to about 80 nucleosides, modified nucleosides, monomers of formula I and/or mimetics in length. One of ordinary skill in the art will appreciate that the invention embodies oligomeric compounds of 8, 9, 10, 11, 12, 113, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleosides and/or modified nucleosides or mimetics in length, or any range therewithin.

In another embodiment, the oligomeric compounds of the invention are 8 to 40 nucleosides, modified nucleosides, monomers of formula I and/or mimetics in length. One having ordinary skill in the art will appreciate that this embodies oligomeric compounds of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleosides and/or modified nucleosides or mimetics in length, or any range therewithin.

In another embodiment, the oligomeric compounds of the invention are 8 to 20 nucleosides, modified nucleosides, monomers of formula I and/or mimetics in length. One having ordinary skill in the art will appreciate that this embodies oligomeric compounds of 8, 9, 10, 11, 12, 13, 14, 15, 16, 17, 18, 19 or 20 nucleosides and/or modified nucleosides or mimetics in length, or any range therewithin.

In another embodiment, the oligomeric compounds of the invention are 12 to 23 nucleosides, modified nucleosides, monomers of formula I and/or mimetics in length. One having ordinary skill in the art will appreciate that this embodies oligomeric compounds of 12, 13, 14, 15, 16, 17, 18, 19, 20, 21, 22 or 23 nucleosides and/or modified nucleosides or mimetics in length, or any range therewithin.

In another embodiment, the oligomeric compounds of the invention are 10 to 16 nucleosides, modified nucleosides, monomers of formula I and/or mimetics in length. One having ordinary skill in the art will appreciate that this embodies oligomeric compounds of 10, 11, 12, 13, 14, 15 or 16 nucleosides and/or modified nucleosides or mimetics in length, or any range therewithin.

In another embodiment, the oligomeric compounds of the invention are 12 to 16 nucleosides, modified nucleosides, monomers of formula I and/or mimetics in length. One having ordinary skill in the art will appreciate that this embodies oligomeric compounds of 12, 13, 14, 15 or 16 nucleosides and/or modified nucleosides or mimetics in length, or any range therewithin.

In another embodiment, the oligomeric compounds of the invention are 10 to 14 nucleosides, modified nucleosides, monomers of formula I and/or mimetics in length. One having ordinary skill in the art will appreciate that this embodies oligomeric compounds of 10, 11, 12, 13 or 14 nucleosides and/or modified nucleosides or mimetics in length, or any range therewithin.

In certain embodiments, the present invention provides oligomeric compounds of any of a variety of ranges of lengths of linked monomeric subunits. In certain embodiments, the invention provides oligomeric compounds consisting of X-Y linked nucleosides, modified nucleosides, monomers of formula I and/or mimetics wherein X and Y are each independently selected from 8, 9, 10, 11, 12, 13, 14, 15, 16, 17, 18, ,19; 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50; provided that X < Y. For example, in certain embodiments, the invention provides oligomeric compounds comprising: 8-9, 8-10, 8-11, 8-12, 8-13, 8-14,-8-15, 8-16, 8-17, 8-18, 8-19, 8-20, 8-21, 8-22, 8-23, 8-24, 8-25, 8-26, 8-27, 8-28, 8-29, 8-30, 9-10, 9-11, 9-12, 9-13, 9-14,,9-15, 9-16, 9-17, 9-18, 9-19, 9-20, 9-21, 9-22, 9-23, 9-24, 9-25, 9-26, 9-27, 9-28, 9-29, 9-30, 10-11, 10-12, 10-13, 10-14, 10-15, 10-16, 10-17, 10-18, 10-19, 10-20, 10-21, 10-22, 10-23, 10-24, 10-25, 10-26, 10-27, 10-28, 10-29, 10-30, 11-12, 11-13, 11-14, 11-15, 11-16, 11-17, 11-18, 11-19, 11-20, 11-21, 11-22, 11-23, 11-24, 11-25, 11-26, 11-27, 11-28, 11-29, 11-30, 12-13, 12-14, 12-15, 12-16, 12-17, 12-18, 12-19, 12-20, 12-21, 12-22, 12-23, 12-24, 12-25, 12-26, 12-27, 12-28, 12-29, 12-30, 13-14, 13-15, 13-16, 13-17, 13-18, 13-19, 13-20, 13-21, 13-22, 13-23, 13-24, 13-25, 13-26, 13-27,13-28, 13-29, 13-30, 14-15, 14-16,14-17, 14-18,14-19,14-20, 14-21, 14-22, 14-23, 14-24, 14-25, 14-26, 14-27, 14-28, 14-29, 14-30, 15-16, 15-17,15-18,15-19,15-20, 15-21, 15-22, 15-23, 15-24, 15-25, 15-26, 15-27, 15-28, 15-29, 15-30, 16-17, 16-18, 16-19, 16-20, 16-21, 16-22, 16-23, 16-24, 16-25, 16-26, 16-27, 16-28,16-29, 16-30, 17-18, 17-19, 17-20, 17-21, 17-22, 17-23, 17-24, 17-25, 17-26, 17-27, 17-28, 17-29, 17-30, 18-19, 18-20, 18-2l, 18-22, 18-23, 18-24, 18-25, 18-26, 18-27, I8-28, 18-29, 18-30, 19-20, 19-21, 19-22, 19-23, 19-24, 19-25, 19-26, 19-29, 19-28, 19-29, 19-30, 20-21, 20-22, 20-23, 20-24, 20-25, 20-26, 20-27, 20-28, 20-29, 20-30, 21-22, 21-23, 21-24, 21-25, 21-26, 21-27, 21-28, 21-29, 21-30, 22-23, 22-24, 22-25, 22-26, 22-27, 22-28, 22-29, 22-30, 23-24, 23-25, 23-26, 23-27, 23-28, 23-29, 23-30, 24-25, 24-26, 24-27, 24-28, 24-29, 24-30, 25-26, 25-27, 25-28, 25-29, 25-30, 26-27, 26-28, 26-29, 26-30, 27-28, 27-29, 27-30, 28-29, 28-30, or 29-30 linked nucleosides, modified nucleosides, monomers of formula I and/or mimetics.

More preferred ranges for the length of the oligomeric compounds in accordance with the present invention are 8-16, 8-40, 10-12, 10-14, 10-16, 10-18, 10-20, 10-21, 12-14, 12-16, 12-18, 12-20 and 12-24 linked nucleosides, modified nucleosides, monomers of formula I and/or mimetics.

Oligomerization of nucleosides, modified nucleosides, monomers of formula I and mimetics, in one aspect of the present invention, is performed according to literature procedures for DNA (Protocols for Oligonucleotides and Analogs, Ed. Agrawal (1993), Humana Press) and/or RNA (Scaringe, Methods (2001), 23, 206-217; Gait et al., Applications of Chemically synthesized RNA in RNA:Protein Interactions, Ed. Smith (1998), 1-36; Gallo et al., Tetrahedron (2001), 57, 5707-5713) synthesis as appropriate. Additional methods for solid-phase synthesis may be found in Caruthers U.S. Patents Nos. 4,415,732; 4,458,066; 4,500,707; 4,668,777; 4,973,679; and 5,132,418; and Koster U.S. Patents Nos. 4,725,677 and Re. 34,069.

Commercially available equipment routinely used for the support medium based synthesis of oligomeric compounds and related compounds is sold by several vendors including, for example, Applied Biosystems (Foster City, CA). Any other means for such synthesis known in the art may additionally or alternatively be employed. Suitable solid phase techniques, including automated synthesis techniques, are described in F. Eckstein (ed.), Oligonucleotides and Analogues, a Practical Approach, Oxford University Press, New York (1991).

The synthesis of RNA and related analogs relative to the synthesis of DNA and related analogs has been increasing as efforts in RNAi increase. The primary RNA synthesis strategies that are presently being used commercially include 5'-O-DMT-2'-O-t-butyldimethylsilyl (TBDMS), 5'-O-DMT-2'-O-[1(2-fluorophenyl)-4-methoxypiperidin-4-yl] (FPMP), 2'-O-[(triisopropylsilyl)oxy]methyl (2'-OCH₂-OSi(iPr)₃ (TOM), and the 5'-O-silyl ether-2'-ACE (5'-O-bis(trimethylsiloxy)cyclododecyloxysilyl ether (DOD)-2'-O bis(2-acetoxyethoxy)methyl (ACE). A current list of some of the major companies currently offering RNA products include Pierce Nucleic Acid Technologies, Dharmacon Research Inc., Ameri Biotechnologies Inc., and Integrated DNA Technologies, Inc. One company, Princeton Separations, is marketing an RNA synthesis activator advertised to reduce coupling times especially with TOM and TBDMS chemistries. Such an activator would also be amenable to the present invention.

The primary groups being used for commercial RNA synthesis are:
TBDMS = 5'-O-DMT-2'-O-t-butyldimethylsilyl;
TOM= 2'-O[(triisopropylsilyl)oxy]methyl;
FPMP = 5'-O-DMT-2'-O-[1(2-fluorophenyl)-4-methoxypiperidin-4-yl]; and
DOD/ACE = (5'-O-bis(trimethylsiloxy)cyclododecyloxysilyl ether-2'-O-bis(2-acetoxyethoxy)methyl.

All of the aforementioned RNA synthesis strategies are amenable to the present invention. Strategies that would be a hybrid of the above e.g. using a 5'-protecting group from one strategy with a 2'-Oprotecting from another strategy is also amenable to the present invention.

In the context of this invention, "hybridization" means pairing of complementary strands which includes pairs of oligomeric compounds or an oligomeric compound and a target nucleic acid such as a mRNA. In the present invention, one mechanism of pairing involves hydrogen bonding, which may be Watson-Crick, Hoogsteen or reversed Hoogsteen hydrogen bonding, between complementary heterocyclic bases. For example, adenine and thymine are complementary nucleobases which pair through the formation of hydrogen bonds. Hybridization can occur under varying circumstances.

An oligomeric compound is specifically hybridizable when binding of the compound to the target nucleic acid interferes with the normal function of the target nucleic acid to cause a loss of activity, and there is a sufficient degree of complementarity to avoid non-specific binding of the oligomeric compound to non-target nucleic acid sequences under conditions in which specific binding is desired, i.e., under physiological conditions in the case *of in vivo* assays or therapeutic treatment, and under conditions in which assays are performed in the case of *in vitro* assays.

"Complementary," as used herein, refers to the capacity for precise pairing of two nucleobases regardless of where the two are located. For example, if a nucleobase at a certain position of an oligomeric compound is capable of hydrogen bonding with a nucleobase at a certain position of a target nucleic acid, the target nucleic acid being a DNA, RNA, or oligonucleotide molecule, then the position of hydrogen bonding between the oligonucleotide and the target nucleic acid is considered to be a complementary position. The oligomeric compound and the further DNA, RNA, or oligonucleotide molecule are complementary to each other when a sufficient number of complementary positions in each molecule are occupied by nucleobases which can hydrogen bond with each other. Thus, "specifically hybridizable" and "complementary" are terms which are used to indicate a sufficient degree of precise pairing or complementarity over a sufficient number of nucleobases such that stable and specific binding occurs between the oligonucleotide and a target nucleic acid.

It is understood in the art that the sequence of an oligomeric compound need not be 100% complementary to that of its target nucleic acid to be specifically hybridizable. Moreover, an oligonucleotide may hybridize over one or more segments such that intervening or adjacent segments are not involved in the hybridization event (e.g., a loop structure or hairpin structure). The oligomeric compounds of the present invention can comprise at least about 70%, at least about 80%, at least about 90%, at least about 95%, or at least about 99% sequence complementarity to a target region within the target nucleic acid sequence to which they are targeted. For example, an oligomeric compound in which 18 of 20 nucleobases of the oligomeric compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an oligomeric compound which is 18 nucleobases in length having 4 (four) noncomplementary nucleobases which are flanked by two regions of complete complementarity with the target nucleic acid would have 77.8% overall complementarity with the target nucleic acid and would thus fall within the scope of the present invention. Percent complementarity of an oligomeric compound with a region of a target nucleic acid can be determined routinely using BLAST programs (basic local alignment search tools) and PowerBLAST programs known in the art (Altschul et al., J. Mol. Biol., 1990, 215, 403-410; Zhang and Madden, Genome Res., 1997, 7, 649-656).

Further included in the present invention are oligomeric compounds such as antisense oligomeric compounds, antisense oligonucleotides, ribozymes, external guide sequence (EGS) oligonucleotides, alternate splicers, primers, probes, and other oligomeric compounds which hybridize to at least a portion of the target nucleic acid. As such, these oligomeric compounds may be introduced in the form of single-stranded, double-stranded, circular or hairpin oligomeric compounds and may contain structural elements such as internal or terminal bulges or loops. Once introduced to a system, the oligomeric compounds of the invention may elicit the action of one or more enzymes or structural proteins to effect modification of the target nucleic acid.

One non-limiting example of such an enzyme is RNAse H, a cellular endonuclease which cleaves the RNA strand of an RNA:DNA duplex. It is known in the art that single-stranded oligomeric compounds which are "DNA-like" elicit RNAse H. Activation of RNase H, therefore, results in cleavage of the RNA target, thereby greatly enhancing the efficiency of oligonucleotide-mediated inhibition of gene expression. Similar roles have been postulated for other ribonucleases such as those in the RNase III and ribonuclease L family of enzymes.

As used herein, the term "antisense compound" includes oligomeric compounds that are at least partially complementary and preferably fully complementary to a target nucleic acid molecule to which it hybridizes. In certain embodiments, an antisense compound modulates (increases or decreases) expression or amount of a target nucleic acid. In certain embodiments, an antisense compound alters splicing of a target pre-mRNA resulting in a different splice variant. Antisense compounds include, but are not limited to, compounds that are oligonucleotides, oligonucleosides, oligonucleotide analogs, oligonucleotide mimetics, and chimeric combinations of these. In one aspect the antisense oligomeric compounds are paired with a sense oligomeric compound to form double stranded duplexes which effect activity through an RNAi mechanism. Consequently, while all antisense compounds are oligomeric compounds, not all oligomeric compounds are antisense compounds.

While one form of oligomeric compound is a single-stranded antisense oligonucleotide, in many species the introduction of double-stranded structures, such as double-stranded RNA (dsRNA) molecules, has been shown to induce potent and specific antisense-mediated reduction of the function of a gene or its associated gene products. This phenomenon occurs in both plants and animals and is believed to have an evolutionary connection to viral defense and transposon silencing.

In some embodiments, "suitable target segments" may be employed in a screen for additional oligomeric compounds that modulate the expression of a selected protein. "Modulators" are those oligomeric compounds that decrease or increase the expression of a nucleic acid molecule encoding a protein and which comprise at least an 8-nucleobase portion which is complementary to a suitable target segment. The screening method comprises the steps of contacting a suitable target segment of a nucleic acid molecule encoding a protein with one or more candidate modulators, and selecting for one or more candidate modulators which decrease or increase the expression of a nucleic acid molecule encoding a protein. Once it is shown that the candidate modulator or modulators are capable of modulating (e.g. either decreasing or increasing) the expression of a nucleic acid molecule encoding a peptide, the modulator may then be employed in further investigative studies of the function of the peptide, or for use as a research, diagnostic, or therapeutic agent in accordance with the present invention.

The suitable target segments of the present invention may also be combined with their respective complementary antisense oligomeric compounds of the present invention to form stabilized double-stranded (duplexed) oligonucleotides. Such double stranded oligonucleotide moieties have been shown in the art to modulate target expression and regulate translation as well as RNA processsing via an antisense mechanism. Moreover, the double-stranded moieties may be subject to chemical modifications (Fire et al., Nature, 1998, 391, 806-811; Timmons and Fire, Nature 1998, 395, 854; Timmons et al., Gene, 2001, 263, 103-112; Tabara et al., Science, 1998, 282, 430-431; Montgomery et al., Proc. Natl. Acad. Sci. USA, 1998, 95, 15502-15507; Tuschl et al., Genes Dev., 1999, 13, 3191-3197; Elbashir et al., Nature, 2001, 41 1, 494-498; Elbashir et al., Genes Dev. 2001, 15, 188-200). For example, such double-stranded moieties have been shown to inhibit the target by the classical hybridization of antisense strand of the duplex to the target, thereby triggering enzymatic degradation of the target (Tijsterman et al., Science, 2002, 295, 694-697).

The oligomeric compounds of the present invention can also be applied in the areas of drug discovery and target validation. The present invention comprehends the use of the oligomeric compounds and targets identified herein in drug discovery efforts to elucidate relationships that exist between proteins and a disease state, phenotype, or condition. These methods include detecting or modulating a target peptide comprising contacting a sample, tissue, cell, or organism with the oligomeric compounds of the present invention, measuring the nucleic acid or protein level of the target and/or a related phenotypic or chemical endpoint at some time after treatment, and optionally comparing the measured value to a non-treated sample or sample treated with a further oligomeric compound of the invention. These methods can also be performed in parallel or in combination with other experiments to determine the function of unknown genes for the process of target validation or to determine the validity of a particular gene product as a target for treatment or prevention of a particular disease, condition, or phenotype.

As used herein, the term "dose" refers to a specified quantity of a pharmaceutical agent provided in a single administration. In certain embodiments, a dose may be administered in two or more boluses, tablets, or injections. For example, in certain embodiments, where subcutaneous administration is desired, the desired dose requires a volume not easily accommodated by a single injection. In such embodiments, two or more injections may be used to achieve the desired dose. In certain embodiments, a dose may be administered in two or more injections to minimize injection site reaction in an individual.

In certain embodiments, chemically-modified oligomeric compounds of the invention may have a higher affinity for target RNAs than does non-modified DNA. In certain such embodiments, higher affinity in turn provides increased potency allowing for the administration of lower doses of such compounds, reduced potential for toxicity, improvement in therapeutic index and decreased overall cost of therapy.

Effect of nucleoside modifications on RNAi activity is evaluated according to existing literature (Elbashir et al., Nature (2001), 411, 494-498; Nishikura et al., Cell (2001), 107, 415-416; and Bass et al., Cell (2000), 101,235-238.)

The oligomeric compounds of the present invention can be utilized for diagnostics, therapeutics, prophylaxis and as research reagents and kits. Furthermore, antisense oligonucleotides, which are able to inhibit gene expression with exquisite specificity, are often used by those of ordinary skill to elucidate the function of particular genes or to distinguish between functions of various members of a biological pathway. The oligomeric compounds of the present invention, either alone or in combination with other oligomeric compounds or therapeutics, can be used as tools in differential and/or combinatorial analyses to elucidate expression patterns of a portion or the entire complement of genes expressed within cells and tissues. Oligomeric compounds can also be effectively used as primers and probes under conditions favoring gene amplification or detection, respectively. These primers and probes are useful in methods requiring the specific detection of nucleic acid molecules encoding proteins and in the amplification of the nucleic acid molecules for detection or for use in further studies. Hybridization of the antisense oligonucleotides, particularly the primers and probes, of the invention with a nucleic acid can be detected by means known in the art. Such means may include conjugation of an enzyme to the oligonucleotide, radiolabelling of the oligonucleotide or any other suitable detection means. Kits using such detection means for detecting the level of selected proteins in a sample may also be prepared.

As one nonlimiting example, expression patterns within cells or tissues treated with one or more oligomeric compounds are compared to control cells or tissues not treated with oligomeric compounds and the patterns produced are analyzed for differential levels of gene expression as they pertain, for example, to disease association, signaling pathway, cellular localization, expression level, size, structure or function of the genes examined. These analyses can be performed on stimulated or unstimulated cells and in the presence or absence of other compounds and or oligomeric compounds which affect expression patterns.

Examples of methods of gene expression analysis known in the art include DNA arrays or microarrays (Brazma and Vilo, FEBS Lett., 2000, 480, 17-24; Celis, et al., FEBS Lett., 2000, 480, 2-16), SAGE (serial analysis of gene expression)(Madden, et al., Drug Discov. Today, 2000, 5, 415-425), READS (restriction enzyme amplification of digested cDNAs) (Prashar and Weissman, Methods Enzymol., 1999, 303, 258-72), TOGA (total gene expression analysis) (Sutcliffe, et al., Proc. Natl. Acad. Sci. U. S. A., 2000, 97, 1976-81), protein arrays and proteomics (Celis, et al., FEBS Lett., 2000, 480, 2-16; Jungblut, et al., Electrophoresis, 1999, 20, 2100-10), expressed sequence tag (EST) sequencing (Celis, et al., FEBS Lett., 2000, 480, 2-16; Larsson, et al., J. Biotechnol., 2000, 80, 143-57), subtractive RNA fingerprinting (SuRF) (Fuchs, et al., Anal. Biochem., 2000, 286,91-98; Larson, et al., Cytometry, 2000, 41, 203-208), subtractive cloning, differential display (DD) (Jurecic and Belmont, Curr. Opin. Microbiol., 2000, 3, 316-21), comparative genomic hybridization (Carulli, et al., J. Cell Biochem. Suppl., 1998, 31, 286-96), FISH (fluorescent in situ hybridization) techniques (Going and Gusterson, Eur. J. Cancer, 1999, 35, 1895-904) and mass spectrometry methods (To, Comb. Chem. High Throughput Screen, 2000, 3,235-41).

While the present invention has been described with specificity in accordance with certain of its embodiments, the following examples serve only to illustrate the invention and are not intended to limit the same.

### Examples (General)

¹H and ¹³C NMR spectra were recorded on a 300 MHz and 75 MHz Bruker spectrometer, respectively.

### Example 1

### Synthesis of Nucleoside Phosphoramidites

The preparation of nucleoside phosphoramidites is performed following procedures that are illustrated herein and in the art such as but not limited to US Patent 6,426,220 and published PCT WO 02/36743.

### Example 2

### Oligonucleoside Synthesis

The oligomeric compounds used in accordance with this invention may be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives.

Oligonucleotides: Unsubstituted and substituted phosphodiester (P=O) oligonucleotides can be synthesized on an automated DNA synthesizer (Applied Biosystems model 394) using standard phosphoramidite chemistry with oxidation by iodine.

Phosphorothioates (P=S) are synthesized similar to phosphodiester oligonucleotides with the following exceptions: thiation is effected by utilizing a 10% w/v solution of 3,H-1,2-benzodithiole-3-one 1,1-dioxide in acetonitrile for the oxidation of the phosphite linkages. The thiation reaction step time is increased to 180 sec and preceded by the normal capping step. After cleavage from the CPG column and deblocking in concentrated ammonium hydroxide at 55°C (12-16 hr), the oligonucleotides are recovered by precipitating with greater than 3 volumes of ethanol from a 1 M NH₄OAc solution. Phosphinate oligonucleotides can be prepared as described in U.S. Patent 5,508,270.

Alkyl phosphonate oligonucleotides can be prepared as described in U.S. Patent 4,469,863.

3'-Deoxy-3'-methylene phosphonate oligonucleotides can be prepared as described in U.S. Patents 5,610,289 or 5,625,050.

Phosphoramidite oligonucleotides can be prepared as described in U.S. Patent, 5,256,775 or U.S. Patent 5,366,878.

Alkylphosphonothioate oligonucleotides can be prepared as described in published PCT applications PCT/US94/00902 and PCT/US93/06976 (published as WO 94/17093 and WO 94/02499, respectively).

3'-Deoxy-3'-amino phosphoramidate oligonucleotides can be prepared as described in U.S. Patent 5,476,925.

Phosphotriester oligonucleotides can be prepared as described in U.S. Patent 5,023,243.

Borano phosphate oligonucleotides can be prepared as described in U.S. Patents 5,130,302 and 5,177,198.

Oligonucleosides: Methylenemethylimino linked oligonucleosides, also identified as MMI linked oligonucleosides, methylenedimethylhydrazo linked oligonucleosides, also identified as MDH linked oligonucleosides, and methylenecarbonylamino linked oligonucleosides, also identified as amide-3 linked oligonucleosides, and methyleneaminocarbonyl linked oligonucleosides, also identified as amide-4 linked oligonucleosides, as well as mixed backbone oligomeric compounds having, for instance, alternating MMI and P=O or P=S linkages can be prepared as described in U.S. Patents 5,378,825, 5,386,023, 5,489,677, 5,602,240 and 5,610,289.

Formacetal and thioformacetal linked oligonucleosides can be prepared as described in U.S. Patents 5,264,562 and 5,264,564.

Ethylene oxide linked oligonucleosides can be prepared as described in U.S. Patent 5,223,618.

### Example 3

### Oligonucleotide Isolation

After cleavage from the controlled pore glass solid support and deblocking in concentrated ammonium hydroxide at 55°C for 12-16 hours, the oligonucleotides or oligonucleosides are recovered by precipitation out of 1 M NH₄OAc with >3 volumes of ethanol. Synthesized oligonucleotides are analyzed by electrospray mass spectroscopy (molecular weight determination) and by capillary gel electrophoresis. The relative amounts of phosphorothioate and phosphodiester linkages obtained in the synthesis is determined by the ratio of correct molecular weight relative to the -16 amu product (+/-32 +/-48). For some studies oligonucleotides are purified by HPLC, as described by Chiang et,al., J. Biol. Chem. 1991, 266, 18162-18171. Results obtained with HPLC-purified material are generally similar to those obtained with non-HPLC purified material.

### Example 4

### Oligonucleotide Synthesis - 96 Well Plate Format

Oligonucleotides can be synthesized via solid phase P(III) phosphoramidite chemistry on an automated synthesizer capable of assembling 96 sequences simultaneously in a 96-well format. Phosphodiester internucleotide linkages are afforded by oxidation with aqueous iodine. Phosphorothioate internucleotide linkages are generated by sulfurization utilizing 3,H-1,2 benzodithiole-3-one 1,1 dioxide (Beaucage Reagent) in anhydrous acetonitrile. Standard base-protected beta-cyanoethyl-diiso-propyl phosphoramidites are purchased from commercial vendors (e.g. PE-Applied Biosystems, Foster City, CA, or Pharmacia, Piscataway, NJ). Non-standard nucleosides are synthesized as per standard or patented methods. They are utilized as base protected beta-cyanoethyldiisopropyl phosphoramidites.

Oligonucleotides are cleaved from support and deprotected with concentrated NH₄OH at elevated temperature (55-60°C) for 12-16 hours and the released product then dried *in vacuo.* The dried product is then re-suspended in sterile water to afford a master plate from which all analytical and test plate samples are then diluted utilizing robotic pipettors.

### Example 5

### Oligonucleotide Analysis using 96-Well Plate Format

The concentration of oligonucleotide in each well is assessed by dilution of samples and UV absorption spectroscopy. The full-length integrity of the individual products is evaluated by capillary electrophoresis (CE) in either the 96-well format (Beckman P/ACE™ MDQ) or, for individually prepared samples, on a commercial CE apparatus (e.g., Beckman P/ACE™ 5000, ABI 270). Base and backbone composition is confirmed by mass analysis of the oligomeric compounds utilizing electrospray-mass spectroscopy. All assay test plates are diluted from the master plate using single and multi-channel robotic pipettors. Plates are judged to be acceptable if at least 85% of the oligomeric compounds on the plate are at least 85% full length.

### Example 6

### Cell Culture and Oligonucleotide Treatment

The effect of oligomeric compounds on target nucleic acid expression is tested in any of a variety of cell types provided that the target nucleic acid is present at measurable levels. This can be routinely determined using, for example, PCR or Northern blot analysis. Cell lines derived from multiple tissues and species can be obtained from American Type Culture Collection (ATCC, Manassas, VA).

The following cell type is provided for illustrative purposes, but other cell types can be routinely used, provided that the target is expressed in the cell type chosen. This can be readily determined by methods routine in the art, for example Northern blot analysis, ribonuclease protection assays or RT-PCR.

b.END cells: The mouse brain endothelial cell line b.END was obtained from Dr. Werner Risau at the Max Plank Institute (Bad Nauheim, Germany). b.END cells were routinely cultured in DMEM, high glucose (Invitrogen Life Technologies, Carlsbad, CA) supplemented with 10% fetal bovine serum (Invitrogen Life Technologies, Carlsbad, CA). Cells were routinely passaged by trypsinization and dilution when they reached approximately 90% confluence. Cells were seeded into 96-well plates (Falcon-Primaria #353872, BD Biosciences, Bedford, MA) at a density of approximately 3000 cells/well for uses including but not limited to oligomeric compound transfection experiments.

Experiments involving treatment of cells with oligomeric compounds:
When cells reach appropriate confluency, they are treated with oligomeric compounds using a transfection method as described.

### LIPOFECTIN™

When cells reached 65-75% confluency, they are treated with oligonucleotide. Oligonucleotide is mixed with LIPOFECTIN™ Invitrogen Life Technologies, Carlsbad, CA) in Opti-MEM™-1 reduced serum medium (Invitrogen Life Technologies, Carlsbad, CA) to achieve the desired concentration of oligonucleotide and a LIPOFECTIN™ concentration of 2.5 or 3 µg/mL per 100 nM oligonucleotide. This transfection mixture is incubated at room temperature for approximately 0.5 hours. For cells grown in 96-well plates, wells are washed once with 100 µL OPTI-MEM™-1 and then treated with 130 µL of the transfection mixture. Cells grown in 24-well plates or other standard tissue culture plates are treated similarly, using appropriate volumes of medium and oligonucleotide. Cells are treated and data are obtained in duplicate or triplicate. After approximately 4-7 hours of treatment at 37°C, the medium containing the transfection mixture is replaced with fresh culture medium. Cells are harvested 16-24 hours after oligonucleotide treatment.

Other suitable transfection reagents known in the art include, but are not limited to, CYTOFECTIN™, LIPOFECTAMINE™, OLIGOFECTAMINE™, and FUGENE™. Other suitable transfection methods known in the art include, but are not limited to, electroporation.

### Example 7

### Real-time Quantitative PCR Analysis of target mRNA Levels

Quantitation of a target mRNA levels was accomplished by real-time quantitative PCR using the ABI PRISM™ 7600, 7700, or 7900 Sequence Detection System (PE-Applied Biosystems, Foster City, CA) according to manufacturer's instructions. This is a closed-tube, non-gel-based, fluorescence detection system which allows high-throughput quantitation of polymerase chain reaction (PCR) products in real-time. As opposed to standard PCR in which amplification products are quantitated after the PCR is completed, products in real-time quantitative PCR are quantitated as they accumulate. This is accomplished by including in the PCR reaction an oligonucleotide probe that anneals specifically between the forward and reverse PCR primers, and contains two fluorescent dyes. A reporter dye (e.g., FAM or JOE, obtained from either PE-Applied Biosystems, Foster City, CA, Operon Technologies Inc., Alameda, CA or Integrated DNA Technologies Inc., Coralville, IA) is attached to the 5' end of the probe and a quencher dye (e.g., TAMRA, obtained from either PE-Applied Biosystems, Foster City, CA, Operon Technologies Inc., Alameda, CA or Integrated DNA Technologies Inc., Coralville, IA) is attached to the 3' end of the probe. When the probe and dyes are intact, reporter dye emission is quenched by the proximity of the 3' quencher dye. During amplification, annealing of the probe to the target sequence creates a substrate that can be cleaved by the 5'-exonuclease activity of Taq polymerase. During the extension phase of the PCR amplification cycle, cleavage of the probe by Taq polymerase releases the reporter dye from the remainder of the probe (and hence from the quencher moiety) and a sequence-specific fluorescent signal is generated. With each cycle, additional reporter dye molecules are cleaved from their respective probes, and the fluorescence intensity is monitored at regular intervals by laser optics built into the ABI PRISM™ Sequence Detection System. In each assay, a series of parallel reactions containing serial dilutions of mRNA from untreated control samples generates a standard curve that is used to quantitate the percent inhibition after antisense oligonucleotide treatment of test samples.

Prior to quantitative PCR analysis, primer-probe sets specific to the target gene being measured are evaluated for their ability to be "multiplexed" with a GAPDH amplification reaction. In multiplexing, both the target gene and the internal standard gene GAPDH are amplified concurrently in a single sample. In this analysis, mRNA isolated from untreated cells is serially diluted. Each dilution is amplified in the presence of primer-probe sets specific for GAPDH only, target gene only ("single-plexing"), or both (multiplexing). Following PCR amplification, standard curves of GAPDH and target mRNA signal as a function of dilution are generated from both the single-plexed and multiplexed samples. If both the slope and correlation coefficient of the GAPDH and target signals generated from the multiplexed samples fall within 10% of their corresponding values generated from the single-plexed samples, the primer-probe set specific for that target is deemed multiplexable. Other methods of PCR are also known in the art.

RT and PCR reagents were obtained from Invitrogen Life Technologies (Carlsbad, CA). RT, real-time PCR was carried out by adding 20 µL PCR cocktail (2.5x PCR buffer minus MgCl₂, 6.6 mM MgCl₂, 375 µM each of dATP, dCTP, dCTP and dGTP, 375 nM each of forward primer and reverse primer, 125 nM of probe, 4 Units RNAse inhibitor, 1.25 Units PLATINUM® Taq, 5 Units MuLV reverse transcriptase, and 2.5x ROX dye) to 96-well plates containing 30 µL total RNA solution (20-200 ng). The RT reaction was carried out by incubation for 30 minutes at 48°C. Following a 10 minute incubation at 95°C to activate the PLATINUM® Taq, 40 cycles of a two-step PCR protocol were carried out: 95°C for 15 seconds (denaturation) followed by 60°C for 1.5 minutes (annealing/extension).

Gene target quantities obtained by RT, real-time PCR are normalized using either the expression level of GAPDH, a gene whose expression is constant, or by quantifying total RNA using RIBOGREEN^{™} (Molecular Probes, Inc. Eugene, OR). GAPDH expression is quantified by real time RT-PCR, by being run simultaneously with the target, multiplexing, or separately. Total RNA is quantified using RiboGreen^{™} RNA quantification reagent (Molecular Probes, Inc. Eugene, OR). Methods of RNA quantification by RIBOGREEN^{™} are taught in Jones, L.J., et al, (Analytical Biochemistry, 1998, 265, 368-374).

In this assay, 170 µL of RIBOGREEN^{™} working reagent (RIBOGREEN^{™} reagent diluted 1:350 in 10mM Tris-HCl, 1 mM EDTA, pH 7.5) is pipetted into a 96-well plate containing 30 µL purified, cellular RNA. The plate is read in a CytoFluor 4000 (PE Applied Biosystems) with excitation at 485nm and emission at 530nm.

### Example 8

### Analysis of oligonucleotide inhibition of a target expression

Antisense modulation of a target expression can be assayed in a variety of ways known in the art. For example, a target mRNA levels can be quantitated by, e.g., Northern blot analysis, competitive polymerase chain reaction (PCR), or real-time PCR. Real-time quantitative PCR is presently desired. RNA analysis can be performed on total cellular RNA or poly(A)+ mRNA. One method of RNA analysis of the present invention is the use of total cellular RNA as described in other examples herein. Methods of RNA isolation are well known in the art. Northern blot analysis is also routine in the art. Real-time quantitative (PCR) can be conveniently accomplished using the commercially available ABI PRISM™ 7600, 7700, or 7900 Sequence Detection System, available from PE-Applied Biosystems, Foster City, CA and used according to manufacturer's instructions.

Protein levels of a target can be quantitated in a variety of ways well known in the art, such as immunoprecipitation, Western blot analysis (immunoblotting), enzyme-linked immunosorbent assay (ELISA) or fluorescence-activated cell sorting (FACS). Antibodies directed to a target can be identified and obtained from a variety of sources, such as the MSRS catalog of antibodies (Aerie Corporation, Birmingham, MI), or can be prepared via conventional monoclonal or polyclonal antibody generation methods well known in the art. Methods for preparation of polyclonal antisera are taught in, for example, Ausubel, F.M. et al., Current Protocols in Molecular Biology, Volume 2, pp. 11.12.1-11.12.9, John Wiley & Sons, Inc., 1997. Preparation of monoclonal antibodies is taught in, for example, Ausubel, F.M. et al., Current Protocols in Molecular Biology, Volume 2, pp. 11.4.1-11.11.5, John Wiley & Sons, Inc., 1997.

Immunoprecipitation methods are standard in the art and can be found at, for example, Ausubel, F.M. et al., Current Protocols in Molecular Biology, Volume 2, pp. 10.16.1-10.16.11, John Wiley & Sons, Inc., 1998. Western blot (immunoblot) analysis is standard in the art and can be found at, for example, Ausubel, F.M. et al., Current Protocols in Molecular Biology, Volume 2, pp. 10.8.1-10.8.21, John Wiley & Sons, Inc., 1997. Enzyme-linked immunosorbent assays (ELISA) are standard in the art and can be found at, for example, Ausubel, F.M. et al., Current Protocols in Molecular Biology, Volume 2, pp. 11.2.1-11.2.22, John Wiley & Sons, Inc., 1991.

### Example 9

### Design of phenotypic assays and in vivo studies for the use of target inhibitors

### Phenotypic assays

Once target inhibitors have been identified by the methods disclosed herein, the oligomeric compounds are further investigated in one or more phenotypic assays, each having measurable endpoints predictive of efficacy in the treatment of a particular disease state or condition.

Phenotypic assays, kits and reagents for their use are well known to those skilled in the art and are herein used to investigate the role and/or association of a target in health and disease. Representative phenotypic assays, which can be purchased from any one of several commercial vendors, include those for determining cell viability, cytotoxicity, proliferation or cell survival (Molecular Probes, Eugene, OR; PerkinElmer, Boston, MA), protein-based assays including enzymatic assays (Panvera, LLC, Madison, WI; BD Biosciences, Franklin Lakes, NJ; Oncogene Research Products, San Diego, CA), cell regulation, signal transduction, inflammation, oxidative processes and apoptosis (Assay Designs Inc., Ann Arbor, MI), triglyceride accumulation (Sigma-Aldrich, St. Louis, MO), angiogenesis assays, tube formation assays, cytokine and hormone assays and metabolic assays (Chemicon International Inc., Temecula, CA; Amersham Biosciences, Piscataway, NJ).

In one non-limiting example, cells determined to be appropriate for a particular phenotypic assay (i.e., MCF-7 cells selected for breast cancer studies; adipocytes for obesity studies) are treated with a target inhibitors identified from the *in vitro* studies as well as control compounds at optimal concentrations which are determined by the methods described above. At the end of the treatment period, treated and untreated cells are analyzed by one or more methods specific for the assay to determine phenotypic outcomes and endpoints.

Phenotypic endpoints include changes in cell morphology over time or treatment dose as well as changes in levels of cellular components such as proteins, lipids, nucleic acids, hormones, saccharides or metals. Measurements of cellular status which include pH, stage of the cell cycle, intake or excretion of biological indicators by the cell, are also endpoints of interest.

Measurement of the expression of one or more of the genes of the cell after treatment is also used as an indicator of the efficacy or potency of the a target inhibitors. Hallmark genes, or those genes suspected to be associated with a specific disease state, condition, or phenotype, are measured in both treated and untreated cells.

### In vivo studies

The individual subjects of the *in vivo* studies described herein are warm-blooded vertebrate animals, which includes humans.

### Example 10

### RNA Isolation

### Poly(A)+ mRNA isolation

Poly(A)+ mRNA is isolated according to Miura et al., (Clin. Chem., 1996, 42, 1758-1764). Other methods for poly(A)+ mRNA isolation are routine in the art. Briefly, for cells grown on 96-well plates, growth medium is removed from the cells and each well is washed with 200 µL cold PBS. 60 µL lysis buffer (10 mM Tris-HCl, pH 7.6, 1 mM EDTA, 0.5 M NaCl, 0.5% NP-40, 20 mM vanadyl-ribonucleoside complex) is added to each well, the plate is gently agitated and then incubated at room temperature for five minutes. 55 µL of lysate is transferred to Oligo d(T) coated 96-well plates (AGCT Inc., Irvine CA). Plates are incubated for 60 minutes at room temperature, washed 3 times with 200 µL of wash buffer (10 mM Tris-HCl pH 7.6, 1 mM EDTA, 0.3 M NaCl). After the final wash, the plate is blotted on paper towels to remove excess wash buffer and then air-dried for 5 minutes. 60 µL of elution buffer (5 mM Tris-HCl pH 7.6), preheated to 70°C, is added to each well, the plate is incubated on a 90°C hot plate for 5 minutes, and the eluate is then transferred to a fresh 96-well plate.

Cells grown on 100 mm or other standard plates may be treated similarly, using appropriate volumes of all solutions.

### Total RNA Isolation

Total RNA is isolated using an RNEASY 96™ kit and buffers purchased from Qiagen Inc. (Valencia, CA) following the manufacturer's recommended procedures. Briefly, for cells grown on 96-well plates, growth medium is removed from the cells and each well is washed with 200 µL cold PBS. 150 µL Buffer RLT is added to each well and the plate vigorously agitated for 20 seconds. 150 µL of 70% ethanol is then added to each well and the contents mixed by pipetting three times up and down. The samples are then transferred to the RNEASY 96™ well plate attached to a QIAVAC™ manifold fitted with a waste collection tray and attached to a vacuum source. Vacuum is applied for 1 minute. 500 µL of Buffer RW1 is added to each well of the RNEASY 96™ plate and incubated for 15 minutes and the vacuum is again applied for 1 minute. An additional 500 µL of Buffer RW1 is added to each well of the RNEASY 96™ plate and the vacuum is applied for 2 minutes. 1 mL of Buffer RPE is then added to each well of the RNEASY 96™ plate and the vacuum applied for a period of 90 seconds. The Buffer RPE wash is then repeated and the vacuum is applied for an additional 3 minutes. The plate is then removed from the QIAVAC™ manifold and blotted dry on paper towels. The plate is then re-attached to the QIAVAC™ manifold fitted with a collection tube rack containing 1.2 mL collection tubes. RNA is then eluted by pipetting 140 µL of RNAse free water into each well, incubating 1 minute, and then applying the vacuum for 3 minutes.

The repetitive pipetting and elution steps may be automated using a QIAGEN Bio-Robot 9604 (Qiagen, Inc., Valencia CA). Essentially, after lysing of the cells on the culture plate, the plate is transferred to the robot deck where the pipetting, DNase treatment and elution steps are carried out.

### Example 11

### Target-specific primers and probes

Probes and primers may be designed to hybridize to a target sequence, using published sequence information.

For example, for human PTEN, the following primer-probe set was designed using published sequence information (GENBANK™ accession number U92436.1, SEQ ID NO: 1).
Forward primer: AATGGCTAAGTGAAGATGACAATCAT (SEQ ID NO: 2)
Reverse primer: TGCACATATCATTACACCAGTTCGT (SEQ ID NO: 3)

And the PCR probe:
FAM-TTGCAGCAATTCACTGTAAAGCTGGAAAGG-TAMRA (SEQ ID NO: 4), where FAM is the fluorescent dye and TAMRA is the quencher dye.

### Example 12

### Western blot analysis of target protein levels

Western blot analysis (immunoblot analysis) is carried out using standard methods. Cells are harvested 16-20 h after oligonucleotide treatment, washed once with PBS, suspended in Laemmli buffer (100 µl/well), boiled for 5 minutes and loaded on a 16% SDS-PAGE gel. Gels are run for 1.5 hours at 150 V, and transferred to membrane for western blotting. Appropriate primary antibody directed to a target is used, with a radiolabeled or fluorescently labeled secondary antibody directed against the primary antibody species. Bands are visualized using a PHOSPHORIMAGER™ (Molecular Dynamics, Sunnyvale CA).

### Example 13

### Preparation of 5'-O-(4,4'-dimethoxytrityl)-5'-(R)-methyl-2'-F-uridine-3'-O-(2-cyanoethyl N,N'-diisopropylamino)phosphoramidite (14)

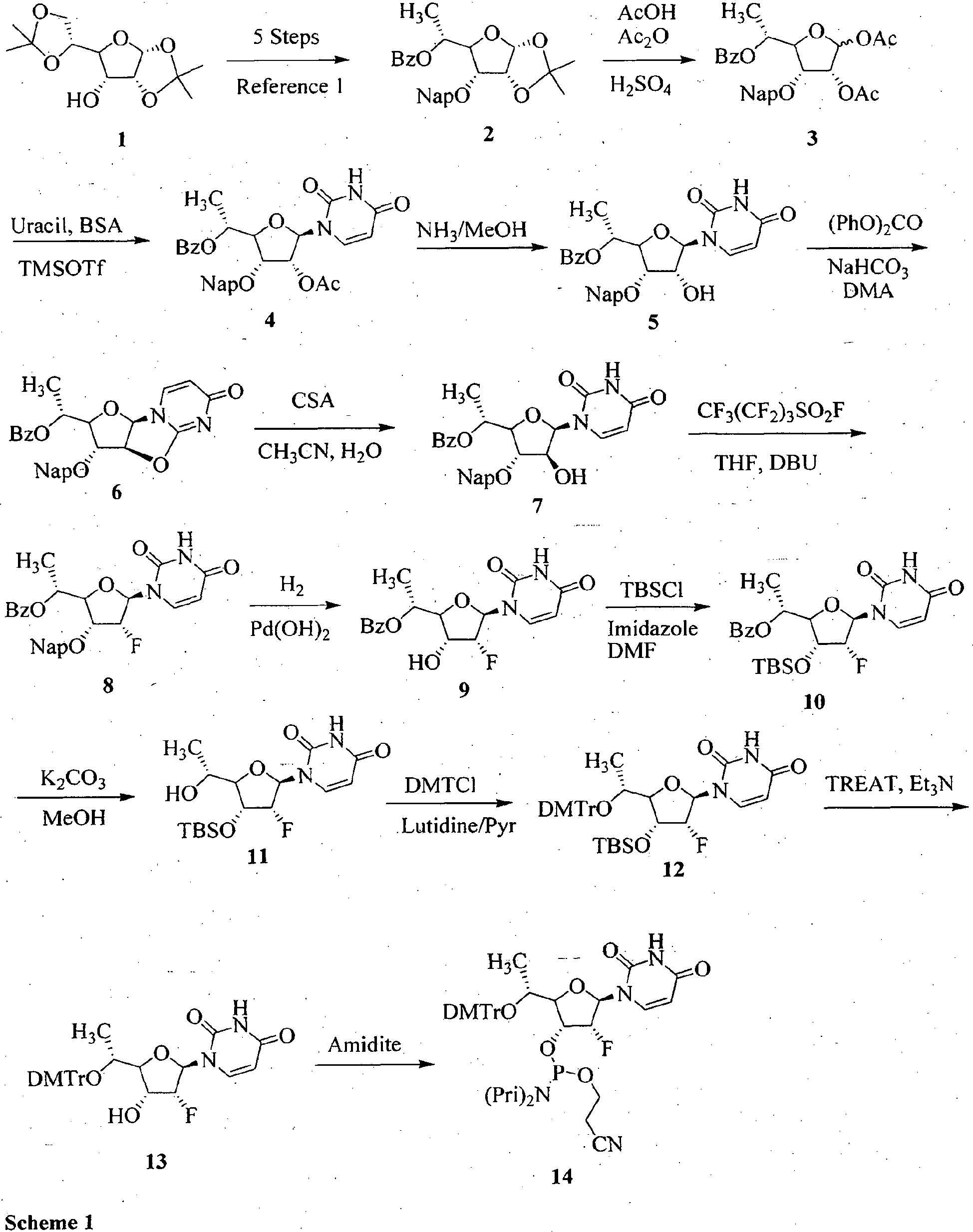

### A) Preparation of Compound 2

Compound 2 is prepared from compound 1 according to the method of De Mesmaeker wherein NapBr is used instead of BnBr ( Reference 1: Amide-Modified Oligonucleotides with Preorganized Backbone and Furanose Rings: Highly Increased Thermodynamic Stability of the Duplexes Formed with their RNA and DNA Complements, De Mesmaeker et al., Synlett, 1997, 1287).

### B) Preparation of Compound 3

Compound 2 (35 g, 78 mmoles) was treated with acetic anhydride (23 mL), acetic acid (glacial, 160 mL) and H₂SO₄ (fuming, 200 uL) for 5 hours at room temperature. The reaction was then poured into ice water and extracted with EtOAc. The organic layer was washed sucessively with H₂O NaHCO₃ (until neutral pH), and brine. The organic layer was then dried over Na₂SO₄, filtered and evaporated to dryness under reduced pressure to give a quantitative yield of Compound 3 which was a clear oil and was used directly in the next step. ¹H NMR was consistent with structure.

### C) Preparation of Compound 4

To a dry flask containing Compound 3 (directly used from previous step, 78 mmoles) was added uracil (11.4 g, 101 mmoles) and CH₃CN (200 mL). Bis(trimethylsilyl)acetamide (BSA, 57 mL, 233 mmoles) was added until a clear solution was observed. Trimethylsilyl triflouromethanesulfonate (TMS-OTf, 50 g, 224 mmoles) was then added and the reaction was heated at 85°C for 2.75 h. The reaction was then poured onto EtOAc/H₂O (800 mL, 1:3 ratio), and some NaHCO₃ (sat) was added to clear up the solution. The organic layer was washed with copius amounts of NaHCO₃ (sat), H₂O brine, and the organic layer was then dried overNa₂SO₄, filtered and evaporated to give the crude Compound. This was purified by flash silica gel chromatography using a gradient with EtOAc/hexanes from 50% to 80% to give 25 g of Compound 4. ¹H NMR was consistent with structure.

### D) Preparation of Compound 5

Compound 4 (24 g, 44 mmoles) was treated with 3.5 N NH₃/MeOH (300 mL) for 3 hours, and then the reaction was concentrated to give 22 g of Compound 5 as a foam. This was used directly in the next step without further purification. LC/MS was consistent with structure.

### E) Preparation of Compound 6

Compound 5 (used directly from previous step, 44 mmoles) was treated with diphenyl carbonate (10.3 g, 48 mmoles) and NaHCO₃ (1.5 g) in dimethylacetamide (44 mL) at 105°C for 5.5 hours. The reaction was then cooled to room temperature and poured into EtOAc/H₂O. The aqueous layer was then extracted with portions of dichloromethane and the combined organic layers were evaporated to give Compound 6 as a crude solid. This was used directly in the next step without further purification. LC/MS was consistent with structure.

### F) Preparation of Compound 7

Compound 6 (used directly from previous step, 44 mmoles) was treated with camphorsulfonic acid (3.8 g) in H₂O (150)/CH₃CN (80 mL). The reaction mixture was heated at 80°C for 16 hours and then poured into EtOAc/H₂O. The organic layer was washed withNaHCO₃ (sat), H₂O brine, and the organic layer was then dried over Na₂SO₄, filtered and evaporated to give the crude Compound. This was purified by flash silica gel chromatography, eluting with 3% MeOH/CH₂Cl₂ to give 13 g of Compound 7. ¹H NMR and LCMS were consistent with structure.

### G) Preparation of Compound 8

Compound 7 (12.2 g, 24.3 mmoles) was dissolved in THF (250 mL) and DBU (7.25 mL) was added. To this solution was added the nonafluorobutanesulfonyl flouride (96%, 11 mL) over a 10 minute period. The reaction was allowed to proceed for 1.75 hours, and then the reaction was then poured into EtOAc/H₂O. The organic layer was separated and dried over Na₂SO₄, filtered and evaporated under reduced pressure to give the crude flourinated Compound. This was then purified by flash silica gel chromatography, eluting with 60% EtOAc/hexanes to give 7.1 g of Compound 8. ¹H NMR and LCMS were consistent with structure.

### H) Preparation of Compound 9

Compound 8 (7.0 g, 13.9 mmoles) was hydrogenated at room temperature and 1 atmosphere in MeOH (300 mL) containing a catalytic amount of Pd(OH)₂ for 17 hours. The reaction was then filtered thru celite and evaportated to give 5 g of Compound 9 as a crude solid. This was used directly in the next step without further purification. ¹H NMR and LCMS were consistent with structure.

### I) Preparation of Compound 10

Compound 9 (used directly from previous step, 13.9 mmoles) was treated with TBSCl (4.8 g) and imidazole (7.8 g) in DMF (40 mL). The reaction mixture was stirred for 16 hours and then poured into EtOA_{C}/H₂O. The organic layer was washed with NaHCO₃ (sat), H₂O, brine, and the organic layer was then dried over Na₂SO₄, filtered and evaporated to give 9 g of crude Compound 9 as a white foam. This was used directly in the next step without further purification. ¹H NMR and LCMS were consistent with structure.

### J) Preparation of Compound 11

Compound 10 (used directly from previous step, 13.9 mmoles) was treated with K₂CO₃ (8.7 g) in MeOH (100 mL) for 4 hours. The reaction was then neutralized with AcOH (7mL) and poured into

EtOAc/H₂O. The organic layer was washed with NaHCO₃ (sat), H₂O, brine, and the organic layer was then dried over Na₂SO₄, filtered and evaporated to give crude Compound 10. This Compound was then dissolved in EtOAc and precipitated by the addition of hexanes to give 4.14 g of Compound 11 after collection by filtration. ¹H NMR and LCMS were consistent with structure.

### K) Preparation of Compound 12

4,4'-Dimethoxytrityl chloride (DMTCl) (3.1 g, 8.7 mmol) was added to a solution of Compound 11 (1.1 g, 2.9 mmol) and 2,6-lutidine (1 mL) in pyridine (35 mL). After heating at 45°C for 24h, the reaction was poured into EtOAc and extracted with brine, dried (Na₂SO₄) and concentrated. Purification by flash silica gel chromatography using a gradient with EtOAc/hexanes from 30% to 60% to give 1.6 g of Compound 12 as a yellow foam. ¹H NMR and LCMS were consistent with structure.

### L) Preparation of Compound 13

Triethylamine trihydrofluoride (1.29 mL, 8.0 mmol) was added to a solution of Compound 12 (1 g, 1.5 mmol) and triethylamine (0.45 mL, 3.2 mmol) in THF (9 mL) in a polypropylene tube. After stirring at room temperature for 24h, the reaction was poured into EtOAc and the organic phase was sequentially washed with H₂O saturated NaHCO₃, brine, dried (Na₂SO₄) and concentrated under vacuum. Purification by column chromatography (SiO₂) using a gradient with EtOAc/hexanes from 50% to 70% to gave 0.74 g of Compound 13 as a white foam. ¹H NMR and LCMS were consistent with structure.

### M) Preparation of 5'-O-(4,4'-dimethoxytrityl)-5'-(R)-methyl-2'-F-uridine-3'-O-(2-cyanoethyl N,N-diisopropylamino)phosphoramidite (14)

2-cyanoethyl *N*,*N*-tetraisopropylphosphoamidite (0.6 mL, 1.9 mmol) was added to a solution of Compound 13 (0.72 g, 1.3 mmol), tetrazole (72 mg, 1.0 mmol), *N-*methylimidazole (24 µL, 0.4 mmol) in DMF (6 mL). After stirring for 8h at room temperature, the reaction was poured into EtOAc and the organic phase was washed with 90% brine, brine, dried (Na₂SO₄) and concentrated under vacuum. Purification by column chromatography (SiO₂) using a gradient with EtOAc/hexanes from 60% to 75% to give 0.69 g of Compound 14 as a white solid. ¹H NMR, ³¹P NMR and LCMS were consistent with structure.

### Example 14

### Preparation of 5'-O-(4,4'-dimethoxytrityl)-5'-(S)-methyl-2'-F-uridine-3'-O-(2-cyanoethyl N,N-diisopropylamino)phosphoramidite (19)

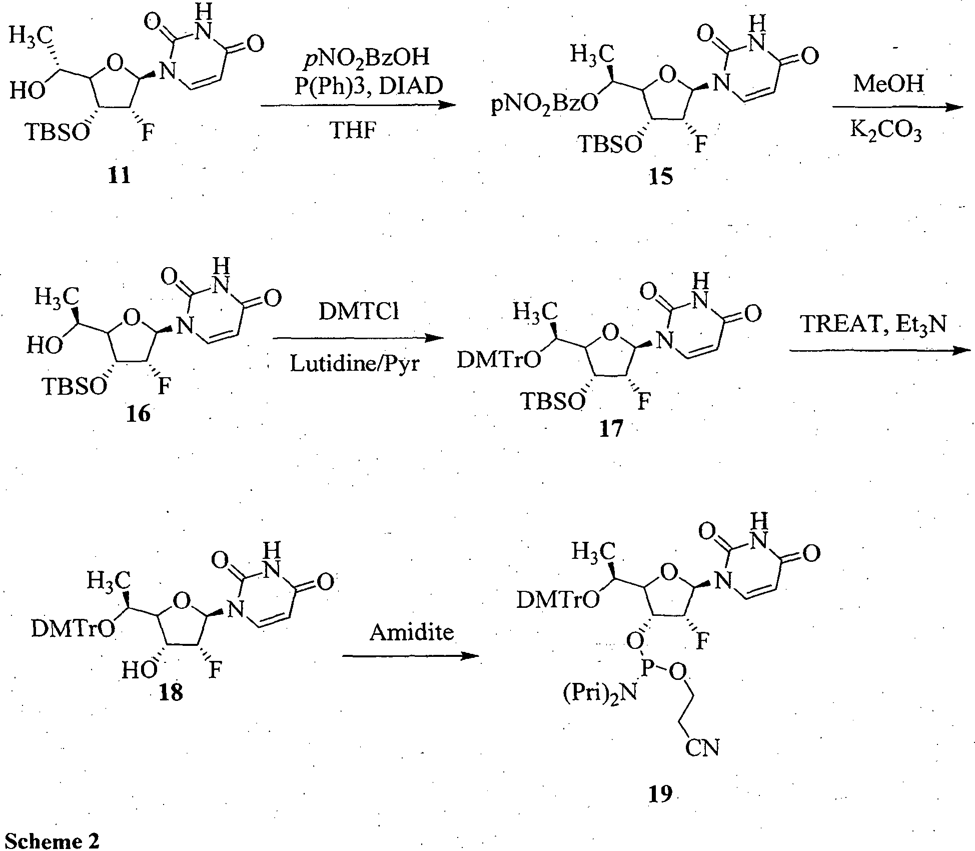

### A) Preparation of Compound 15

To a solution of Compound 11 (2.5 g, 6.68 mmoles), PPh₃ (7 g, 27 mmoles), p-nitrobenzoic acid (4.5 g, 27 mmoles) in THF (55mL) maintained at 0°C was added diisopropylazodicarboxylate (DIAD, 5.2 mL, 27 mmoles). The reaction was then allowed to proceed at room temperature under an inert atmosphere for 16 hours. The reaction was then poured into Et₂O and the organic phase was washed with NaHCO₃ (sat), H₂O brine, dried (Na₂SO₄) and concentrated under vacuum. Purification by flash column chromatography (SiO₂) using a gradient with EtOAc/hexanes from 40% to 85% to give 3.5 g of Compound 15 as a yellow solid. ¹H NMR and LCMS were consistent with structure.

### B) Preparation of Compound 16

Compound 15 (3.5 g, 6.7 mmoles) was treated with K₂CO₃ (3.6 g, 26 mmoles) in MeOH (40 mL) for 1 hour. The reaction was then quenched with AcOH (3 mL) and then poured into EtOAc and the organic phase was washed with H₂O brine, dried (Na₂SO₄) and concentrated under vacuum. The resultant solid was treated with hexanes, collected by filtration, and the resultant solid washed with hexanes and dried to give 2.5 g of Compound 16 as an off-white solid. ¹H NMR and LCMS were consistent with structure.

### C) Preparation of Compound 17

4,4'-Dimethoxytrityl, chloride (DMTCl) (5.7 g, 17 mmol) was added to a solution of Compound 16 (2.0 g, 5.3 mmol) and 2,6-lutidine (1.9 mL) in pyridine (50 mL). After heating at 45°C for 24h, the reaction was poured into EtOAc and extracted with brine, dried (Na₂SO₄) and concentrated. Purification by flash silica gel chromatography using a gradient with EtOAc/hexanes from 33% to 45% to give 3.3 g of Compound 17 as a yellow foam. ¹HNMR and LCMS were consistent with structure.

### D) Preparation of Compound 18

Triethylamine trihydrofluoride (4.9 mL, 30 mmol) was added to a solution of Compound 17 (2.8 g, 4.1 mmol) and triethylamine (2.2 mL, 16 mmol) in THF (25 mL) in a polypropylene tube. After stirring at room temperature for 24h, the reaction was poured into EtOAc/H₂O and the organic phase was sequentially washed with H₂O, saturated NaHCO₃, brine, dried (Na₂SO₄) and concentrated under vacuum. Purification by column chromatography (SiO₂) using a gradient with EtOAc/hexanes from 50% to 70% to give 2.1 g of Compound 18 as a white foam. ¹H NMR and LCMS were consistent with structure.

### E) Preparation of 5'-O-(4,4'-dimethoxytrityl)-5'-(S)-methyl-2'-F-uridine-3'-O-(2-cyanoethyl N,N-diisopropylamino)phosphoramidite 19

2-cyanoethyl *N*,*N*-tetraisopropylphosphoamidite (1.3 mL, 4.1 mmol) was added to a solution of Compound 18 (2.2 g, 3.9 mmol), tetrazole (158 mg, 2.3 mmol), *N-*methylimidazole (58 µL, 0.7 mmol) in DMF (13 mL). After stirring for 8h at room temperature, the reaction was poured into EtOAc/H₂O and the organic phase was washed with 90% brine, brine, dried (Na₂SO₄) and concentrated under vacuum. Purification by column chromatography (SiO₂) using a gradient with EtOAc/hexanes from 60% to 75% to give 2.3 g of Compound 19 as a white solid. ¹H NMR, ³¹P NMR and LCMS were consistent with structure.

### Example 15

### Preparation of 5'-O-(4,4'-dimethoxytrityl)-5'-(R)-methyl-2'-F-N-benzyl cytidine-3'-O-(2-cyanoethyl N,N-diisopropylamino)phosphoramidite 23

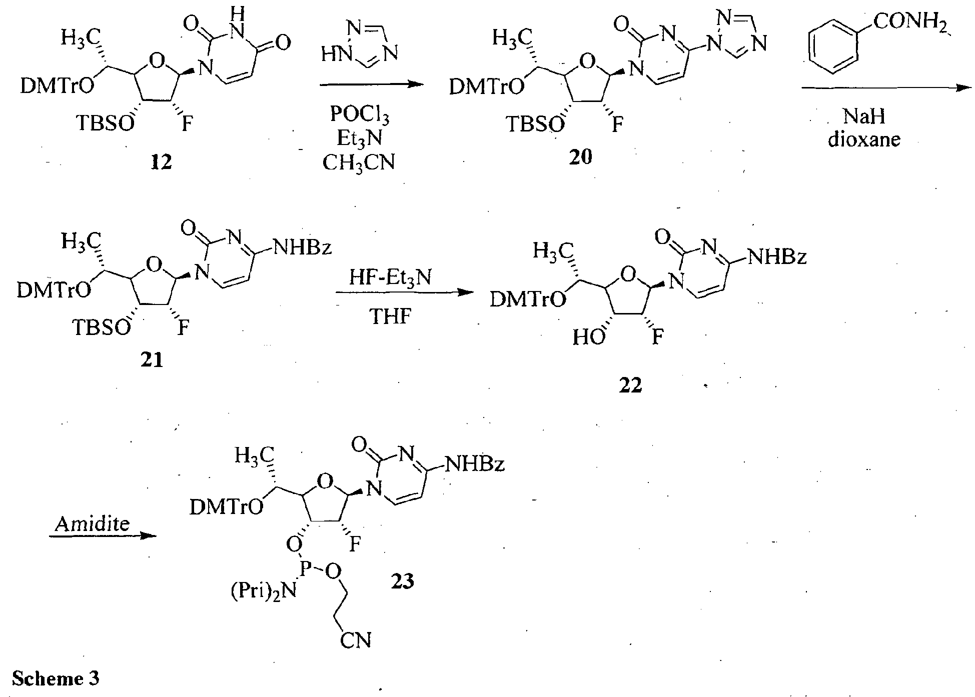

### A) Preparation of Compound 21

To a stirred mixture of 1,2,3-triazole (2.5 g, 51 mmoles) in CH₃CN (25 mL) was added POCl₃ (1.1 mL, 12 mmoles). The reaction was then cooled to 0°C, under an inert atmosphere, and then Et₃N (8.2 mL, 59 mmoles) was added. The thick white suspension was allowed to stir at 0°C for 20 min, and then Compound 12 (1 g, 1.5 mmoles) was added. The reaction was allowed to proceed until the reaction was determined to be complete by TLC (Rf changes from 0.4 to 0.2, 50%EtOAc/hexanes, about 4 hours). The reaction was then poured into EtOAc/H₂O and the organic phase was washed with NaHCO₃, H₂O brine, dried (Na₂SO₄) and concentrated under vacuum to give crude intermediate Compound 20. To this was added a suspension of benzamide (1.4 g, 12 mmoles) and NaH (60% in mineral oil, 474 mg, 12 mmoles) in 1,4-dioxane (14mL) that had been stirring for 30 minutes. After stirring for 1h at, the reaction was poured into EtOAc/H₂O/NH₄Cl and the organic phase was washed with H₂O brine, dried (Na₂SO₄) and concentrated under vacuum. Purification by column chromatography (SiO₂, eluting with 40% EtOAc/hexanes) provided 0.91 g of Compound 21 as a white solid. ¹H NMR and LCMS were consistent with structure.

### B) Preparation of Compound 22

Triethylamine trihydrofluoride (1.29 mL, 8.0 mmol) was added to a solution of Compound 21 (750 mg, 1.1 mmol) and triethylamine (0.45 mL, 3.2 mmol) in THF (9 mL) in a polypropylene tube. After stirring at room temperature for 24h, the reaction was poured into EtOAc/H₂O and the organic phase was sequentially washed with H₂O saturated NaHCO₃, brine, dried (Na₂SO₄) and concentrated under vacuum. Purification by column chromatography (SiO₂) using a gradient with EtOAc/hexanes from 50% to 70% to give 0.55 g of Compound 22 as a white foam. ¹H NMR and LCMS were consistent with structure.

### C) Preparation of 5'-O-(4,4'-dimethoxytrityl)-5'-(IR)-methyl-2'-F-N-benzyl cytidine-3'-O-(2-cyanoethyl N,N-diisopropylamino)phosphoramidite 23

2-cyanoethyl *N*,*N*-tetraisopropylphosphoamidite (0.39 mL, 1.2 mmol) was added to a solution of Compound 22 (0.53 g, 0.82 mmol), tetrazole (46 mg, 0.66 mmol), *N-*methylimidazole (16 µL, 0.2 mmol) in DMF (6 mL). After stirring for 8h at room temperature, the reaction was poured into EtOAc/H₂O and the organic phase was washed with 90% brine, brine, dried (Na₂SO₄) and concentrated under vacuum. Purification by column chromatography (SiO₂) using a gradient with EtOAc/hexanes from 60% to 70% to give 0.68 g of Compound 23 as a white solid. ¹H NMR, ³¹P NMR and LCMS were consistent with structure.

### Example 16

### Preparation of 5'-O-(4,4'-dimethoxytrityl)-5'-(S)-methyl-2'-F-N-benzyl cytidine-3'-O-(2-cyanoethyl N,N-diisopropylamino)phosphoramidite 27

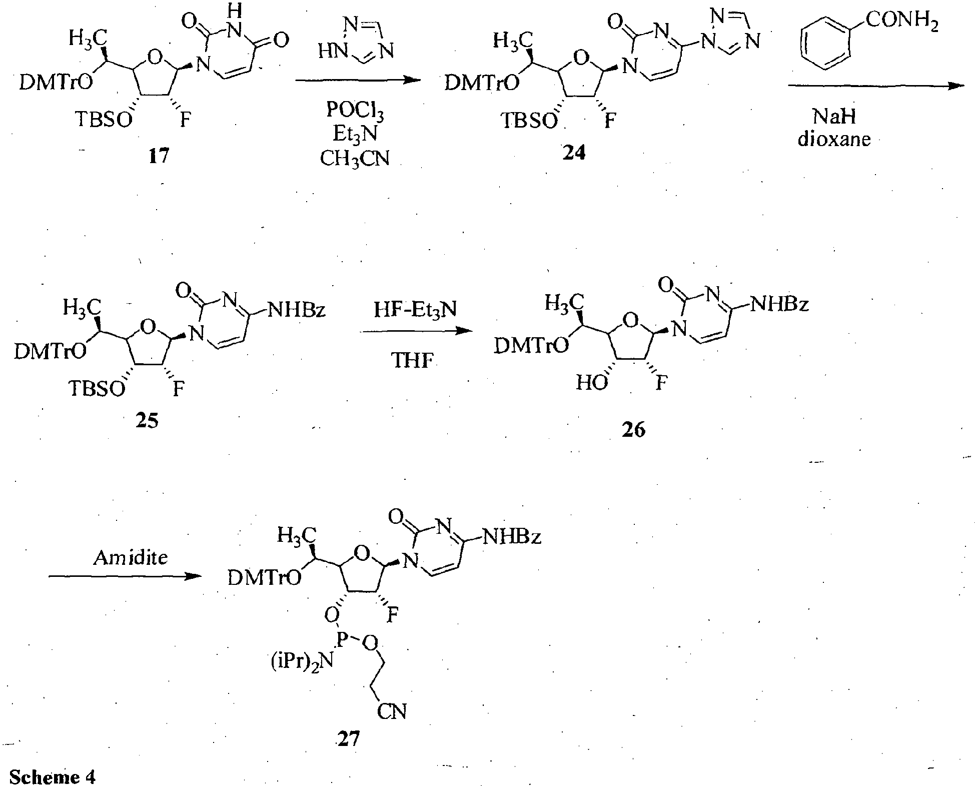

### A) Preparation of Compound 25

To a stirred mixture of 1,2,3-triazole (11.3 g, 231 mmoles) in CH₃CN (80 mL) was added POCl₃ (3.6 mL, 39 mmoles). The reaction was then cooled to 0°C, under an inert atmosphere, and then Et₃N (26.6 mL, 191 mmoles) was added. The thick white suspension was allowed to stir at 0°C for 20 min, and then Compound 17 (3.3 g, 4.8 mmoles) was added. The reaction was allowed to proceed until the reaction was determined to be complete by TLC (Rf changes from 0.4 to 0.2, 50% EtOAc/hexanes, about 4 hours). The reaction was then poured into EtOAc/H₂O and the organic phase was washed with NaHCO₃, H₂O brine, dried (Na₂SO₄) and concentrated under vacuum to give crude intermediate Compound 24. To this was added a suspension of benzamide (3.5 g, 30 mmoles) and NaH (60% in mineral oil, 1.2 g, 30 mmoles) in 1,4-dioxane (14mL) that had been stirring for 30 minutes. After stirring for 1h at room temperature, the reaction was poured into EtOAc/H₂O/NH₄Cl and the organic phase was washed with H₂O brine, dried (Na₂SO₄) and concentrated under vacuum. Purification by column chromatography (SiO₂, eluting with 40% EtOAc/hexanes) provided 0.91 g of Compound 25 as a white solid. ¹H NMR and LCMS were consistent with structure.

### B) Preparation of Compound 26

Triethylamine trihydrofluoride (3.9 mL, 24 mmol) was added to a solution of Compound 25 (3 g, 4.5 mmol) and triethylamine (1.4 mL, 9.6 mmol) in THF (27 mL) in a polypropylene tube. After stirring atroom temperature for 24h, the reaction was poured into EtOAc/H₂O and the organic phase was sequentially washed with H₂O saturated NaHCO₃, brine, dried (Na₂SO₄) and concentrated under vacuum. Purification by column chromatography (SiO₂) using a gradient with EtOAc/hexanes from 50% to 70% to give 2.3 g of Compound 26 as a white foam. ¹H NMR and LCMS were consistent with structure.

### C) Preparation of 5'-O-(4,4'-dimethoxytrityl)-5'-(S)-methyl-2'-F-N-benzyl cytidine-3'-O-(2-cyanoethyl N,N-diisopropylamino)phosphoramidite 27

2-cyanoethyl *N*,*N*-tetraisopropylphosphoamidite (1.6 mL, 3.2 mmol) was added to a solution of Compound 26 (2.2 g, 1.4 mmol), tetrazole (186 mg, 2.7 mmol), *N-*methylimidazole (68 µL, 0.7 mmol) in DMF (15 mL). After stirring for 8h at room temperature, the reaction was poured into EtOAc/H₂O and the organic phase was washed with 90% brine, brine, dried (Na₂SO₄) and concentrated under vacuum. Purification by column chromatography (SiO₂) using a gradient with EtOAc/hexanes from 60% to 75% to give 2.6 g of Compound 27 as a white solid. ¹H NMR, ³¹P NMR and LCMS were consistent with structure.

### Example 17

### 5'-(R)-CH₃-2'-F, 5'-(S)-CH₃-2'-F, 2'-F and 2'-O-MOE 2-10-2 gapped oligomers targeted to PTEN: in vitro study

In accordance with the present invention, oligomeric compounds were synthesized and tested for their ability to inhibit PTEN expression over a range of doses. b.END cells were treated with the 5'-(R)-CH₃-2'-F (392750), 5'-(S)-CH₃-2'-F (392751), 2'-F (392753) and 2'-O-MOE (392752) modified oligomers at a concentration of 20 nM using methods described herein. Expression levels of PTEN were determined using real-time PCR and normalized to RIBOGREEN™ as described in other examples herein. Tm's were assessed in 100 mM phosphate buffer, 0.1 mM EDTA, pH 7, at 260 nm using 4 µM of the respective modified oligomeric compound listed below and 4µM complementary RNA.

| **SEQ ID NO./ISIS NO.** | **Composition (5' to 3')** | **% Inhibition** | **Tm °C** |
|---|---|---|---|
| 05/392750 | C_{R}U_{R}TAGCACTGGCC_{R}U_{R} | 19 | 45.3 |
| 05/392751 | C_{S}U_{S}TAGCACTGGCC_{S}U_{S} | 31 | 50.2 |
| 05/392752 | C_{f}U_{f}TAGCACTGGCC_{f}U_{f} | 23 | 53.4 |
| 05/392753 | CₑUₑTAGCACTGGCCₑUₑ | 31 | 50.8 |

Underlined nucleosides indicate that the internucleoside linkage is a phosphorothioate, subscript R indicates a 5'-(R)-CH₃-2'-F nucleoside, subscript S indicates a 5'-(S)-CH₃-2'-F nucleoside, subscript f indicates a 2'-F nucleoside, subscript e indicates a 2'-O-MOE nucleoside and unsubscripted nucleosides are β-D-2'-deoxyribonucleosides.

### Example 18

### 5'-(R)-CH₃-2'-F, 5'-(S)-CH₃-2'-F, 2'-F and 2'-O-MOE 2-10-2 gapped oligomers targeted to PTEN: in vivo study

Six week old male Balb/c mice (Jackson Laboratory, Bar Harbor, ME) were injected twice weekly for 3 weeks with a 5'-(R or S)-CH₃-2'-F (392750 and 392751 respectively), 2'-F (392753) and 2'-O-MOE (392752) modified oligomers targeted to PTEN at a dose of 0.5 or 2 µmol/kg. The mice were sacrificed 48 hours following the final administration to determine the PTEN mRNA levels in liver using real-time PCR and RIBOGREEN® RNA quantification reagent (Molecular Probes, Inc. Eugene, OR) according to standard protocols. PTEN mRNA levels were determined relative to total RNA (using Robogreen), prior to normalization to saline-treated control. Results are presented in the table below as the average % inhibition of mRNA expression for each treatment group, normalized to saline-injected control.

| **SEQ ID NO./ISIS NO.** | **Composition (5' to 3')** | **Dose µmol/kg** | **% Inhibition** |
|---|---|---|---|
| 05/392750 | C_{R}U_{R}TAGCACTGGCC_{R}U_{R} | 0.5 | 0 |
| | | 2.0 | 24 |
| 05/392751 | C_{S}U_{S}TAGCACTGGCC_{S}U_{S} | 0.5 | 15 |
| | | 2.0 | 30 |
| 05/392752 | C_{f}U_{f}TAGCACTGGCC_{f}U_{f} | 0.5 | 14 |
| | | 2.0 | 16 |
| 05/392753 | CₑUₑTAGCACTGGCCₑUₑ | 0.5 | 10 |
| | | 2.0 | 22 |

Underlined nucleosides indicate that the internucleoside linkage is a phosphorothioate, subscript R indicates a 5'-(R)-CH₃-2'-F nucleoside, subscript S indicates a 5'-(S)-CH₃-2'-F nucleoside, subscript f indicates a 2'-F nucleoside, subscript e indicates a 2'-O-MOE nucleoside and unsubscripted nucleosides are β-D-2'-deoxyribonucleosides.

Liver transaminase levels, alanine aminotranferease (ALT) and aspartate aminotransferase (AST), in serum were also measured relative to saline injected mice. The approximate liver transaminase levels are listed in the table below.

| **SEQ ID NO./ISIS NO.** | **Dose µmol/kg** | **AST** | **ALT** |
|---|---|---|---|
| 05/392750 | 0.5 | 70.8 | 34.0 |
| | 2.0 | 61.8 | 32.8 |
| 05/392751 | 0.5 | 58.0 | 36.8 |
| | 2.0 | 68.5 | 41.5 |
| 05/392752 | 0.5 | 56.5 | 37.5 |
| | 2.0 | 60.8 | 33.5 |
| 05/392753 | 0.5 | 61.5 | 34.8 |
| | 2.0 | 51.0 | 32.0 |

The measured transaminase levels for mice treated with modified oligomers were not elevated to a level indicative of hepatotoxicity.

The effects on liver, spleen and kidney weights were also determined. Signigicant changes in liver, spleen and kidney weight can indicate that a particular compound casuses toxic effects. The data are expressed as percent change in body or organ weight ("+" indicates an increase, "-" indicates a decrease). The results are listed in the table below.

| **SEQ ID NO./ISIS NO.** | **Dose µmol/kg** | **Liver** | **Spleen** | **Kidney** |
|---|---|---|---|---|
| Saline | N/A | 1.00 | 0.98 | 1.01 |
| 05/392750 | 0.5 | 1.04 (+4%) | 1.09 (+11%) | 0.98 (-3%) |
| | 2.0 | 1.11 (+11%) | 1.08 (+10%) | 1.01 (0%) |
| 05/392751 | 0.5 | 1.13 (+13%) | 1.08 (+10%) | 1.04 (+3%) |
| | 2.0 | 1.15 (+15%) | 1.08 (+10%) | 1.05 (+4%) |
| 05/392752 | 0.5 | 1.07 (+7%) | 1.00 (+2%) | 1.05 (+4%) |
| | 2.0 | 1.02 (+2%) | 0.92 (-6%) | 1.02 (+1%) |
| 05/392753 | 0.5 | 1.10 (+10%) | 1.05 (+7%) | 1.07 (+6%) |
| | 2.0 | 1.15 (+15%) | 1.25 (+27%) | 1.08 (+7%). |

### Example 19

### Nuclease stability of 5'-(R or S)-CH₃-2'-F modified oligomers treated with SVPD

The relative nuclease stability of the oligomeric compounds listed below was determined following treatment with snake venom phosphodiesterase (SVPD). The oligomeric compounds, having a 2-10-2 gapped motif, were each prepared as a 500 µL mixture containing: 5 µL IOOµM oligomer, 50 µL phosphodiesterase I @ 0.5 Units/mL in SVPD buffer (50 mM Tris-HcL, pH 7.5, 8 mM MgCl₂) final concentration 0.05 Units/mL, 445 µL SVP buffer. Samples were incubated at 37°C in a water bath. Aliquats (100 µL) were taken at 0, 1, 2 and 4 days with fresh enzyme added at days 1 and 2. EDTA was added to aliquats immediately after removal to quench enzyme activity. Samples were analized on IP HPLC/MS.

| **SEQ ID NO./ISIS NO.** | **Composition (5' to 3')** |
|---|---|
| 05/392750 | C_{R}U_{R}TAGCACTGGCCRU_{R} |
| 05/392751 | C_{S}U_{S}TAGCACTGGCC_{S}U_{S} |
| 05/392752 | C_{f}U_{f}TAGCACTGGCC_{f}U_{f} |
| 05/392753 | CₑUₑTAGCACTGGCCₑUₑ |

Underlined nucleosides indicate that the internucleoside linkage is a phosphorothioate, subscript R indicates a 5'-(R)-CH3-2'-F nucleoside, subscript S indicates a 5'-(S)-CH₃-2'-F nucleoside, subscript f indicates a 2'-F nucleoside, subscript e indicates a 2'-O-MOE nucleoside and unsubscripted nucleosides are β-D-2'-deoxyribonucleosides.

| **SEQ ID NO./ISIS NO.** | **% Composition at 24 hours** | **% Composition at 48 hours** | **% Composition at 96 hours** |
|---|---|---|---|
| 05/392750 | 87% | 72% | 54% |
| 05/392751 | 92% | 68% | 42% |
| 05/392752 | 13% | 6% | 5% |
| 05/392753 | 58% | 46% | 36% |

Oligomeric compounds (392750 and 392751) containing 5'-(R or S)-CH₃-2'-F nucleosides showed a noticeable improvement over the oligomeric compound (392753) containing 2'-O-MOE nucleosides and also a marked improvement over the oligomeric compound (392752) containg 2'-F nucleosides.

## Claims

1. A compound having the formula: 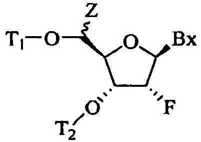 wherein:
Bx is an optionally modified heterocyclic base moiety;
T₁ is H or a hydroxyl protecting group;
T₂ is H, a hydroxyl protecting group or a reactive phosphorus group;
Z is C₁-C₆ alkyl, C₂-C₆ alkenyl, C₂-C₆ alkynyl, substituted C₁-C₆ alkyl, substituted C₂-C₆ alkenyl or substituted C₂-C₆ alkynyl, and
wherein each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

2. The compound of claim 1 wherein Z is methyl, ethyl, vinyl, hydroxymethyl, aminomethylene, methoxymethylene, allyl, or propyl.

3. The compound of any one of claims 1 or 2 wherein Z is methyl.

4. The compound of any one of claims 1 to 3 having the configuration:

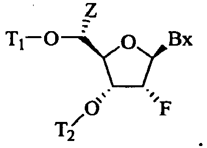

5. The compound of any one of claims 1 to 3 having the configuration:

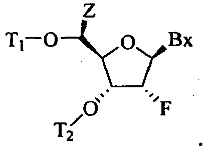

6. The compound of claim 1 wherein at least one of T₁ and T₂ is a hydroxyl protecting group wherein each of said hydroxyl protecting groups is, independently, selected from acetyl, benzyl, benzoyl, 2,6-dichlorobenzyl, t-butyldimethylsilyl, t-butyl-diphenylsilyl, mesylate, tosylate, dimethoxytrityl (DMT), 9-phenylxanthine-9-yl (Pixyl) and 9-(p-methoxyphenyl)xanthine-9-yl (MOX).

7. The compound of any one of claims 1 to 6 wherein T₂ is a reactive phosphorus group selected from diisopropylcyanoethoxy phosphoramidite or H-phosphonate.

8. The compound of any one of claims 1 to 6 wherein said T₁ is 4,4'-dimethoxytrityl and T₂ is diisopropylcyanoethoxy phosphoramidite.

9. The compound of any one of claims 1 to 8 wherein Bx is uracil, 5-methyluracil, 5-thiazolo-uracil, 2-thio-uracil, 5-propynyl-uracil, thymine, 2'-thio-thymine, cytosine, 5-methylcytosine, 5-thiazolo-cytosine, 5-propynyl-cytosine, adenine, guanine or 2,6-diaminopurine.

10. An oligomeric compound comprising at least one monomer having formula I: 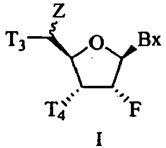 wherein independently for each of said at least one monomer of formula I:
Bx is an optionally modified heterocyclic base moiety;
T₃ is hydroxyl, a protected hydroxyl, a phosphate moiety, a linked conjugate group or an internucleoside linking group attaching said monomer of formula I to a nucleoside, a nucleotide, a monomeric subunit, an oligonucleoside, an oligonucleotide or an oligomeric compound;
each T₄ is, independently, is H, a hydroxyl protecting group, a linked conjugate group or an internucleoside linking group attaching said monomer of formula I to a nucleoside, a nucleotide, a monomeric subunit, an oligonucleoside, an oligonucleotide or an oligomeric compound;
wherein at least one of T₃ and T₄ is an internucleoside linking group attaching said monomer of formula I to a nucleoside, a nucleotide, a monomeric subunit, an oligonucleoside, an oligonucleotide, or an oligomeric compound;
Z is C₁-C₆ alkyl, C₂-C₆ alkenyl, C₂-C₆ alkynyl, substituted C₁-C₆ alkyl, substituted C₂-C₆ alkenyl or substituted C₂-C₆ alkynyl, and
wherein each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

11. The oligomeric compound of claim 10 wherein each Z is, independently, methyl, ethyl, vinyl, hydroxymethyl, aminomethyl, methoxymethyl, allyl, or propyl.

12. The oligomeric compound of claim 10 wherein each Z is methyl.

13. The oligomeric compound of any one of claims 10 to 12 wherein each Bx is, independently selected from uracil, 5-methyluracil, 5-propynyl-uracil, thymine, cytosine, 5-methylcytosine, 5-propynyl-cytosine, adenine and guanine.

14. The oligomeric compound of any one of claims 10 to 13 wherein each monomer of formula I has the configuration:

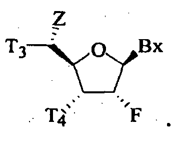

15. The oligomeric compound of any one of claims 10 to 13 wherein each monomer of formula I has the configuration:

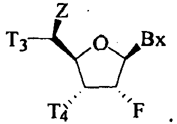

16. The oligomeric compound of any one of claims 10 to 15 wherein one T₃ is H or a hydroxyl protecting group or a phosphate group, substituted phosphate group, phosphorothioate group or a substituted phosphorothioate group.

17. The oligomeric compound of any one of claims 10 to 16 wherein one T₄ is H or a hydroxyl protecting group.

18. i) The oligomeric compound of any one of claims 10 to 17, comprising at least one region of at least two contiguous monomers of formula I, or
ii) the oligomeric compound of any one of claims 10 to 17 comprising at least two regions of at least two contiguous monomers of formula I.

19. The oligomeric compound of claim 18 section ii) comprising a gapped oligomeric compound comprising at least one region of from about 8 to about 14 contiguous β-D-2'-deoxyribofuranosyl nucleosides .

20. The oligomeric compound of claim 19 wherein said region of contiguous β-D-2'-deoxyribofuranosyl nucleosides is from about 8 to about 11 nucleosides.

21. The oligomeric compound of any one of claims 10 to 16 comprising a first region of from 2 to 3 contiguous monomers, an optional third region having 1 monomer or 2 contiguous monomers, and a second region located between said first and said third regions comprising from 8 to 14 β-D-2'-deoxyribofuranosyl nucleosides wherein each of said monomers is a monomer of formula I.

22. The oligomeric compound of claim 21 wherein said second region comprises from 8 to 11 β-D-2'-deoxyribofuranosyl nucleosides, or
the oligomeric compound of claim 21 comprising said third region having 2 contiguous monomers of formula I, or
the oligomeric compound of claim 21 comprising said third region having one monomer of formula I.

23. The oligomeric compound of claim 21 where the Z group for each of said monomers of formula I are in the R configuration, and optionally wherein each Z is methyl.

24. The oligomeric compound of claim 21 where the Z group for each of said monomers of formula I are in the S configuration, and optionally wherein each Z is methyl.

25. The oligomeric compound of claim 10 comprising from about 8 to about 40 nucleosides and/or monomers in length.

26. An in vitro method of reducing target mRNA comprising contacting one or more cells or a tissue with an oligomeric compound of claim 10 or
an oligomeric compound of claim 10 for use in an in vivo method of reducing target mRNA comprising contacting one or more cells, a tissue or an animal with an oligomeric compound of claim 10..

27. A composition comprising first and second chemically synthesized oligomeric compounds, wherein:
the first oligomeric compound is fully complementary to the second oligomeric compound;
the first oligomeric compound is fully complementary to a selected nucleic acid target;
at least one of said first and second oligomeric compounds comprises at least one monomer having formula I: 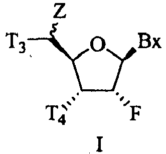
wherein independently for each of said at least one monomer of formula I:
Bx is an optionally modified heterocyclic base moiety;
T₃ is hydroxyl, a protected hydroxyl, a phosphate moiety, a linked conjugate group or an internucleoside linking group attaching said monomer of formula I to a nucleoside, a nucleotide, a monomeric subunit, an oligonucleoside, an oligonucleotide or an oligomeric compound;
each T₄ is, independently, is H, a hydroxyl protecting group, a linked conjugate group or an internucleoside linking group attaching said monomer of formula I to a nucleoside, a nucleotide, a monomeric subunit, an oligonucleoside, an oligonucleotide or an oligomeric compound;
wherein at least one of T₃ and T₄ is an internucleoside linking group attaching said monomer of formula I to a nucleoside, a nucleotide, a monomeric subunit, an oligonucleoside, an oligonucleotide, or an oligomeric compound;
Z is C₁-C₆ alkyl, C₂-C₆ alkenyl, C₂-C₆ alkynyl, substituted C₁-C₆ alkyl, substituted C₂-C₆ alkenyl or substituted C₂-C₆ alkynyl, and
wherein each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

28. The composition of claim 27 wherein each of said first and second oligomeric compounds comprises from about 17 to about 26 nucleosides and/or monomers in length.

29. An in vitro method of reducing target mRNA comprising contacting one or more cells or a tissue with a composition of claim 27 or
a composition of claim 27 for use in an in vivo method of reducing target mRNA comprising contacting one or more cells, a tissue or an animal with a composition of claim 27.

## Patentansprüche

1. Verbindung mit der Formel: 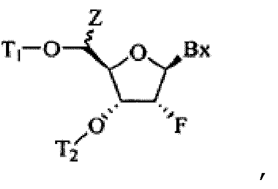 wobei:
Bx für eine gegebenenfalls modifizierte heterocyclische Basengruppierung steht;
T₁ für H oder eine Hydroxyl-Schutzgruppe steht;
T₂ für H, eine Hydroxyl-Schutzgruppe oder eine reaktive Phosphorgruppe steht;
Z für C₁-C₆-Alkyl, C₂-C₆-Alkenyl, C₂-C₆-Alkinyl, substituiertes C₁-C₆-Alkyl, substituiertes C₂-C₆-Alkenyl oder substituiertes C₂-C₆-Alkinyl steht und wobei die substituierten Gruppen jeweils unabhängig ein- oder mehrfach mit gegebenenfalls geschützten Substituentengruppen substituiert sind, die unabhängig aus Halogen, Oxo, Hydroxyl, OJ₁, NJ₁J₂, SJ₁, N₃, OC(=X)J₁, OC(=X)NJ₁J₂, NJ₃C(=X)NJ₁J₂ und CN ausgewählt sind, wobei J₁, J₂ und J₃ jeweils unabhängig für H oder C₁-C₆-Alkyl stehen und X für 0, S oder NJ₁ steht.

2. Verbindung nach Anspruch 1, wobei Z für Methyl, Ethyl, Vinyl, Hydroxymethyl, Aminomethylen, Methoxymethylen, Allyl oder Propyl steht.

3. Verbindung nach Anspruch 1 oder 2, wobei Z für Methyl steht.

4. Verbindung nach einem der Ansprüche 1 bis 3 mit der Konfiguration:

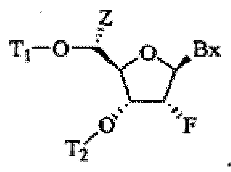

5. Verbindung nach einem der Ansprüche 1 bis 3 mit der Konfiguration:

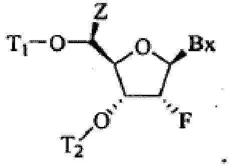

6. Verbindung nach Anspruch 1, wobei T₁ oder/und T₂ für eine Hydroxyl-Schutzgruppe steht bzw. stehen, wobei die Hydroxyl-Schutzgruppen jeweils unabhängig aus Acetyl, Benzyl, Benzoyl, 2,6-Dichlorbenzyl, t-Butyldimethylsilyl, t-Butyldiphenylsilyl, Mesylat, Tosylat, Dimethoxytrityl (DMT), 9-Phenylxanthin-9-yl (Pixyl) und 9-(p-Methoxyphenyl)xanthin-9-yl (MOX) ausgewählt sind.

7. Verbindung nach einem der Ansprüche 1 bis 6, wobei T₂ für eine aus Diisopropylcyanoethoxyphosphoramidit oder H-Phosphonat ausgewählte reaktive Phosphorgruppe steht.

8. Verbindung nach einem der Ansprüche 1 bis 6, wobei das T₁ für 4,4'-Dimethoxytrityl und T₂ für Diisopropylcyanoethoxyphosphoramidit steht.

9. Verbindung nach einem der Ansprüche 1 bis 8, wobei Bx für Uracil, 5-Methyluracil, 5-Thiazolouracil, 2-Thiouracil, 5-Propinyluracil, Thymin, 2'-Thiothymin, Cytosin, 5-Methylcytosin, 5-Thiazolocytosin, 5-Propinylcytosin, Adenin, Guanin oder 2,6-Diaminopurin steht.

10. Oligomere Verbindung, umfassend wenigstens ein Monomer mit der Formel I: 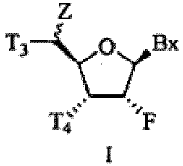 wobei unabhängig für jedes wenigstens eine Monomer der Formel I:
Bx für eine gegebenenfalls modifizierte heterocyclische Basengruppierung steht;
T₃ für Hydroxyl, ein geschütztes Hydroxyl, eine Phosphatgruppierung, eine verknüpfte Konjugatgruppe oder eine Internukleosid-Verknüpfungsgruppe, durch die das Monomer der Formel I an ein Nukleosid, ein Nukleotid, eine Monomer-Untereinheit, ein Oligonukleosid, ein Oligonukleotid oder eine oligomere Verbindung gebunden ist, steht;
T₄ jeweils unabhängig für H, eine Hydroxyl-Schutzgruppe, eine verknüpfte Konjugatgruppe oder eine Internukleosid-Verknüpfungsgruppe, durch die das Monomer der Formel I an ein Nukleosid, ein Nukleotid, eine Monomer-Untereinheit, ein Oligonukleosid, ein Oligonukleotid oder eine oligomere Verbindung gebunden ist, steht;
wobei T₃ oder/und T₄ für eine Internukleosid-Verknüpfungsgruppe, durch die das Monomer der Formel I an ein Nukleosid, ein Nukleotid, eine Monomer-Untereinheit, ein Oligonukleosid, ein Oligonukleotid oder eine oligomere Verbindung gebunden ist, steht bzw. stehen;
Z für C₁-C₆-Alkyl, C₂-C₆-Alkenyl, C₂-C₆-Alkinyl, substituiertes C₁-C₆-Alkyl, substituiertes C₂-C₆-Alkenyl oder substituiertes C₂-C₆-Alkinyl steht und wobei die substituierten Gruppen jeweils unabhängig ein- oder mehrfach mit gegebenenfalls geschützten Substituentengruppen substituiert sind, die unabhängig aus Halogen, Oxo, Hydroxyl, OJ₁, NJ₁J₂, SJ₁, N₃, OC(=X)J₁, OC(=X)NJ₁J₂, NJ₃C(=X)NJ₁J₂ und CN ausgewählt sind, wobei J₁, J₂ und J₃ jeweils unabhängig für H oder C₁-C₆-Alkyl stehen und X für 0, S oder NJ₁ steht.

11. Oligomere Verbindung nach Anspruch 10, wobei Z jeweils unabhängig für Methyl, Ethyl, Vinyl, Hydroxymethyl, Aminomethyl, Methoxymethyl, Allyl oder Propyl steht.

12. Oligomere Verbindung nach Anspruch 10, wobei Z jeweils für Methyl steht.

13. Oligomere Verbindung nach einem der Ansprüche 10 bis 12, wobei Bx jeweils unabhängig aus Uracil, 5-Methyluracil, 5-Propinyluracil, Thymin, Cytosin, 5-Methylcytosin, 5-Propinylcytosin, Adenin und Guanin ausgewählt ist.

14. Oligomere Verbindung nach einem der Ansprüche 10 bis 13, wobei jedes Monomer der Formel I die Konfiguration: 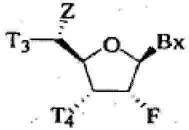 aufweist.

15. Oligomere Verbindung nach einem der Ansprüche 10 bis 13, wobei jedes Monomer der Formel I die Konfiguration: 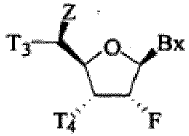 aufweist.

16. Oligomere Verbindung nach einem der Ansprüche 10 bis 15, wobei ein T₃ für H oder eine Hydroxyl-Schutzgruppe oder für eine Phosphatgruppe, substituierte Phosphatgruppe, Phosphorothioatgruppe oder eine substituierte Phosphorothioatgruppe steht.

17. Oligomere Verbindung nach einem der Ansprüche 10 bis 16, wobei ein T₄ für H oder eine Hydroxyl-Schutzgruppe steht.

18. i) Oligomere Verbindung nach einem der Ansprüche 10 bis 17, umfassend wenigstens einen Bereich von wenigstens zwei zusammenhängenden Monomeren der Formel I, oder
ii) oligomere Verbindung nach einem der Ansprüche 10 bis 17, umfassend wenigstens zwei Bereiche von wenigstens zwei zusammenhängenden Monomeren der Formel I.

19. Oligomere Verbindung nach Anspruch 18, Abschnitt ii), umfassend eine oligomere Verbindung mit Lücke, umfassend wenigstens einen Bereich von etwa 8 bis etwa 14 zusammenhängenden β-D-2'-Desoxyribofuranosyl-Nukleosiden.

20. Oligomere Verbindung nach Anspruch 19, wobei es sich bei dem Bereich von zusammenhängenden β-D-2'-Desoxyribofuranosyl-Nukleosiden um etwa 8 bis etwa 11 Nukleoside handelt.

21. Oligomere Verbindung nach einem der Ansprüche 10 bis 16, umfassend einen ersten Bereich von 2 bis 3 zusammenhängenden Monomeren, einen fakultativen dritten Bereich mit 1 Monomer oder 2 zusammenhängenden Monomeren und einen zwischen dem ersten und dem dritten Bereich liegenden zweiten Bereich, umfassend 8 bis 14 β-D-2'-Desoxyribofuranosyl-Nukleoside, wobei es sich bei den Monomeren jeweils um ein Monomer der Formel I handelt.

22. Oligomere Verbindung nach Anspruch 21, wobei der zweite Bereich 8 bis 11 β-D-2'-Desoxyribofuranosyl-Nukleoside umfasst, oder
oligomere Verbindung nach Anspruch 21, umfassend den dritten Bereich mit 2 zusammenhängenden Monomeren der Formel I, oder
oligomere Verbindung nach Anspruch 21, umfassend den dritten Bereich mit einem Monomer der Formel I.

23. Oligomere Verbindung nach Anspruch 21, in der die Z-Gruppe bei den Monomeren der Formel I jeweils in der R-Konfiguration vorliegen, und gegebenenfalls wobei Z jeweils für Methyl steht.

24. Oligomere Verbindung nach Anspruch 21, in der die Z-Gruppe bei den Monomeren der Formel I jeweils in der S-Konfiguration vorliegen, und gegebenenfalls wobei Z jeweils für Methyl steht.

25. Oligomere Verbindung nach Anspruch 10, umfassend eine Länge von etwa 8 bis etwa 40 Nukleosiden und/oder Monomeren.

26. In-vitro-Verfahren zur Reduzierung von Ziel-mRNA, bei dem eine oder mehrere Zellen oder ein Gewebe mit einer oligomeren Verbindung nach Anspruch 10 in Kontakt gebracht wird bzw. werden, oder oligomere Verbindung nach Anspruch 10 zur Verwendung bei einem In-vivo-Verfahren zur Reduzierung von Ziel-mRNA, bei dem eine oder mehrere Zellen, ein Gewebe oder ein Tier mit einer oligomeren Verbindung nach Anspruch 10 in Kontakt gebracht wird bzw. werden.

27. Zusammensetzung, umfassend erste und zweite chemisch synthetisierte oligomere Verbindungen, wobei:
die erste oligomere Verbindung vollständig komplementär zur zweiten oligomeren Verbindung ist;
die erste oligomere Verbindung vollständig komplementär zu einem ausgewählten Nukleinsäureziel ist;
die erste oder/und zweite oligomere Verbindung wenigstens ein Monomer mit der Formel I: 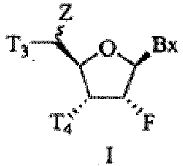 umfasst bzw. umfassen, wobei unabhängig für jedes wenigstens eine Monomer der Formel I:
Bx für eine gegebenenfalls modifizierte heterocyclische Basengruppierung steht;
T₃ für Hydroxyl, ein geschütztes Hydroxyl, eine Phosphatgruppierung, eine verknüpfte Konjugatgruppe oder eine Internukleosid-Verknüpfungsgruppe, durch die das Monomer der Formel I an ein Nukleosid, ein Nukleotid, eine Monomer-Untereinheit, ein Oligonukleosid, ein Oligonukleotid oder eine oligomere Verbindung gebunden ist, steht;
T₄ jeweils unabhängig für H, eine Hydroxyl-Schutzgruppe, eine verknüpfte Konjugatgruppe oder eine Internukleosid-Verknüpfungsgruppe, durch die das Monomer der Formel I an ein Nukleosid, ein Nukleotid, eine Monomer-Untereinheit, ein Oligonukleosid, ein Oligonukleotid oder eine oligomere Verbindung gebunden ist, steht;
wobei T₃ oder/und T₄ für eine Internukleosid-Verknüpfungsgruppe, durch die das Monomer der Formel I an ein Nukleosid, ein Nukleotid, eine Monomer-Untereinheit, ein Oligonukleosid, ein Oligonukleotid oder eine oligomere Verbindung gebunden ist, steht bzw. stehen;
Z für C₁-C₆-Alkyl, C₂-C₆-Alkenyl, C₂-C₆-Alkinyl, substituiertes C₁-C₆-Alkyl, substituiertes C₂-C₆-Alkenyl oder substituiertes C₂-C₆-Alkinyl steht und wobei die substituierten Gruppen jeweils unabhängig ein- oder mehrfach mit gegebenenfalls geschützten Substituentengruppen substituiert sind, die unabhängig aus Halogen, Oxo, Hydroxyl, OJ₁, NJ₁J₂, SJ₁, N₃, OC(=X)J₁, OC(=X)NJ₁J₂, NJ₃C(=X)NJ₁J₂ und CN ausgewählt sind, wobei J₁, J₂ und J₃ jeweils unabhängig für H oder C₁-C₆-Alkyl stehen und X für 0, S oder NJ₁ steht.

28. Zusammensetzung nach Anspruch 27, wobei die ersten und zweiten oligomeren Verbindungen jeweils eine Länge von etwa 17 bis etwa 26 Nukleosiden und/oder Monomeren umfassen.

29. In-vitro-Verfahren zur Reduzierung von Ziel-mRNA, bei dem eine oder mehrere Zellen oder ein Gewebe mit einer Zusammensetzung nach Anspruch 27 in Kontakt gebracht wird bzw. werden, oder Zusammensetzung nach Anspruch 27 zur Verwendung bei einem In-vivo-Verfahren zur Reduzierung von Ziel-mRNA, bei dem eine oder mehrere Zellen, ein Gewebe oder ein Tier mit einer Zusammensetzung nach Anspruch 27 in Kontakt gebracht wird bzw. werden.

## Revendications

1. Composé ayant la formule : 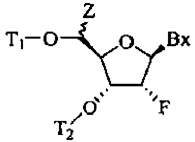 dans lequel :
Bx est un fragment de base hétérocyclique facultativement modifié ;
T₁ est H ou un groupe protecteur d'hydroxyle ;
T₂ est H, un groupe protecteur d'hydroxyle ou un groupe à phosphore réactif ;
Z est alkyle en C₁-C₆" alcényle en C₂-C₆, alcynyle en C₂-C₆, alkyle en C₁-C₆ substitué, alcényle en C₂-C₆ substitué ou alcynyle en C₂-C₆ substitué, et
où chacun des groupes substitués, est, indépendamment, mono- ou poly-substitué par des groupes substituants facultativement protégés indépendamment choisis parmi halogène, oxo, hydroxyle, OJ₁, NJ₁J₂, SJ₁, N₃, OC(=X)J₁, OC(=X)NJ₁J₂, NJ₃C(=X)NJ₁J₂ et CN, où chaque J₁, J₂ et J₃ est, indépendamment, H ou alkyle en C₁-C₆" et X est 0, S ou NJ₁.

2. Composé de la revendication 1 dans lequel Z est méthyle, éthyle, vinyle, hydroxyméthyle, aminométhylène, méthoxyméthylène, allyle, ou propyle.

3. Composé de l'une quelconque des revendications 1 ou 2 dans lequel Z est méthyle.

4. Composé de l'une quelconque des revendications 1 à 3 ayant la configuration :

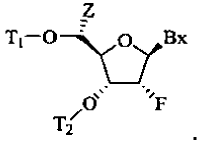

5. Composé de l'une quelconque des revendications 1 à 3 ayant la configuration :

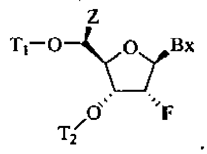

6. Composé de la revendication 1 dans lequel au moins l'un de T₁ et T₂ est un groupe protecteur d'hydroxyle dans lequel chacun desdits groupes protecteurs d'hydroxyle est, indépendamment, choisi parmi acétyle, benzyle, benzoyle, 2,6-dichlorobenzyle, t-butyldiméthylsilyle, t-butyl-diphénylsilyle, mésylate, tosylate, diméthoxytrityle (DMT), 9-phénylxanthine-9-yle (Pixyl) et 9-(p-méthoxyphényl)xanthine-9-yle (MOX).

7. Composé de l'une quelconque des revendications 1 à 6 dans lequel T₂ est un groupe à phosphore réactif choisi parmi diisopropylcyanoéthoxyphosphoramidite ou H-phosphonate.

8. Composé de l'une quelconque des revendications 1 à 6 dans lequel ledit T₁ est 4,4'-diméthoxytrityle et T₂ est diisopropylcyanoéthoxyphosphoramidite.

9. Composé de l'une quelconque des revendications 1 à 8 dans lequel Bx est uracile, 5-méthyluracile, 5-thiazolo-uracile, 2-thio-uracile, 5-propynyl-uracile, thymine, 2'-thio-thymine, cytosine, 5-méthylcytosine, 5-thiazolo-cytosine, 5-propynyl-cytosine, adénine, guanine ou 2,6-diaminopurine.

10. Composé oligomère comprenant au moins un monomère ayant la formule I : 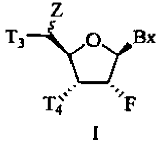 dans lequel, indépendamment pour chacun dudit au moins un monomère de formule I :
Bx est un fragment de base hétérocyclique facultativement modifié ;
T₃ est hydroxyle, un hydroxyle protégé, un fragment phosphate, un groupe conjugué lié ou un groupe de liaison internucléosidique reliant ledit monomère de formule I à un nucléoside, un nucléotide, une sous-unité monomère, un oligonucléoside, un oligonucléotide ou un composé oligomère ;
chaque T₄ est, indépendamment, H, un groupe protecteur d'hydroxyle, un groupe conjugué lié ou un groupe de liaison internucléosidique reliant ledit monomère de formule I à un nucléoside, un nucléotide, une sous-unité monomère, un oligonucléoside, un oligonucléotide ou un composé oligomère ;
où au moins l'un de T₃ et T₄ est un groupe de liaison internucléosidique reliant ledit monomère de formule I à un nucléoside, un nucléotide, une sous-unité monomère, un oligonucléoside, un oligonucléotide, ou un composé oligomère ;
Z est alkyle en C₁-C₆" alcényle en C₂-C₆, alcynyle en C₂-C₆, alkyle en C₁-C₆ substitué, alcényle en C₂-C₆ substitué ou alcynyle en C₂-C₆ substitué, et
où chacun des groupes substitués, est, indépendamment, mono- ou poly-substitué par des groupes substituants facultativement protégés indépendamment choisis parmi halogène, oxo, hydroxyle, OJ₁, NJ₁J₂, SJ₁, N₃, OC(=X)J₁, OC(=X)NJ₁J₂, NJ₃C(=X)NJ₁J₂ et CN, où chaque J₁, J₂ et J₃ est, indépendamment, H ou alkyle en C₁-C₆" et X est 0, S ou NJ₁.

11. Composé oligomère de la revendication 10 dans lequel chaque Z est, indépendamment, méthyle, éthyle, vinyle, hydroxyméthyle, aminométhyle, méthoxyméthyle, allyle, ou propyle.

12. Composé oligomère de la revendication 10 dans lequel chaque Z est méthyle.

13. Composé oligomère de l'une quelconque des revendications 10 à 12 dans lequel chaque Bx est, indépendamment choisi parmi uracile, 5-méthyluracile, 5-propynyluracile, thymine, cytosine, 5-méthylcytosine, 5-propynyl-cytosine, adénine et guanine.

14. Composé oligomère de l'une quelconque des revendications 10 à 13 dans lequel chaque monomère de formule I a la configuration :

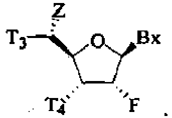

15. Composé oligomère de l'une quelconque des revendications 10 à 13 dans lequel chaque monomère de formule I a la configuration :

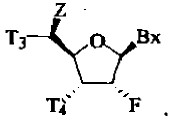

16. Composé oligomère de l'une quelconque des revendications 10 à 15 dans lequel un T₃ est H ou un groupe protecteur d'hydroxyle ou un groupe phosphate, un groupe phosphate substitué, un groupe phosphorothioate ou un groupe phosphorothioate substitué.

17. Composé oligomère de l'une quelconque des revendications 10 à 16 dans lequel un T₄ est H ou un groupe protecteur d'hydroxyle.

18. i) Composé oligomère de l'une quelconque des revendications 10 à 17, comprenant au moins one région d'au moins deux monomères de formule I contigus, ou
ii) composé oligomère de l'une quelconque des revendications 10 à 17 comprenant au moins deux régions d'au moins deux monomères de formule I contigus.

19. Composé oligomère de la revendication 18 section ii) comprenant un composé oligomère à brèche comprenant au moins une région d'environ 8 à environ 14 β-D-2'-désoxyribofuranosyl-nucléosides contigus.

20. Composé oligomère de la revendication 19 dans lequel ladite région de β-D-2'-désoxyribofuranosyl-nucléosides contigus est d'environ 8 à environ 11 nucléosides.

21. Composé oligomère de l'une quelconque des revendications 10 à 16 comprenant une première région de 2 à 3 monomères contigus, une troisième région facultative ayant 1 monomère ou 2 monomères contigus, et une deuxième région située entre ladite première et ladite troisième régions comprenant de 8 à 14 β-D-2'-désoxyribofuranosyl-nucléosides où chacun desdits monomères est un monomère de formule I.

22. Composé oligomère de la revendication 21 dans lequel ladite deuxième région comprend de 8 à 11 β-D-2'-désoxyribofuranosyl-nucléosides, ou
le composé oligomère de la revendication 21 comprenant ladite troisième région ayant 2 monomères de formule I contigus, ou
le composé oligomère de la revendication 21 comprenant ladite troisième région ayant un monomère de formule I.

23. Composé oligomère de la revendication 21 où le groupe Z pour chacun desdits monomères de formule I sont dans la configuration R, et facultativement où chaque Z est méthyle.

24. Composé oligomère de la revendication 21 où le groupe Z pour chacun desdits monomères de formule I est dans la configuration S, et facultativement où chaque Z est méthyle.

25. Composé oligomère de la revendication 10 comprenant d'environ 8 à environ 40 nucléosides et/ou monomères de longueur.

26. Procédé *in vitro* de réduction d'ARNm cible comprenant la mise en contact d'une ou plusieurs cellules ou d'un tissu avec un composé oligomère de la revendication 10 ou
un composé oligomère de la revendication 10 pour utilisation dans un procédé *in vivo* de réduction d'ARNm cible comprenant la mécanisme d'une ou plusieurs cellules, d'un tissu ou d'un animal avec un composé oligomère de la revendication 10.

27. Composition comprenant des premier et deuxième composés oligomères chimiquement synthétisés, dans laquelle :
le premier composé oligomère est totalement complémentaire du deuxième composé oligomère ;
le premier composé oligomère est totalement complémentaire d'un acide nucléique cible sélectionné ;
au moins l'un desdits premier et deuxième composés oligomères comprend au moins un monomère ayant la formule I : 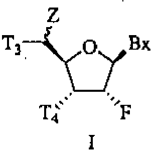 où indépendamment pour chacun desdits au moins un monomère de formule I :
Bx est un fragment de base hétérocyclique facultativement modifié ;
T₃ est hydroxyle, un hydroxyle protégé, un fragment phosphate, un groupe conjugué lié ou un groupe de liaison internucléosidique reliant ledit monomère de formule I à un nucléoside, un nucléotide, une sous-unité monomère, un oligonucléoside, un oligonucléotide ou un composé oligomère ;
chaque T₄ est, indépendamment, H, un groupe protecteur d'hydroxyle, un groupe conjugué lié ou un groupe de liaison internucléosidique reliant ledit monomère de formule I à un nucléoside, un nucléotide, une sous-unité monomère, un oligonucléoside, un oligonucléotide ou un composé oligomère ;
où au moins l'un de T₃ et T₄ est un groupe de liaison internucléosidique reliant ledit monomère de formule I à un nucléoside, un nucléotide, une sous-unité monomère, un oligonucléoside, un oligonucléotide, ou un composé oligomère ;
Z est alkyle en C₁-C₆" alcényle en C₂-C₆, alcynyle en C₂-C₆, alkyle en C₁-C₆ substitué, alcényle en C₂-C₆ substitué ou alcynyle en C₂-C₆ substitué, et
où chacun des groupes substitués, est, indépendamment, mono- ou poly-substitué par des groupes substituants facultativement protégés indépendamment choisis parmi halogène, oxo, hydroxyle, OJ₁, NJ₁J₂, SJ₁, N₃, OC(=X)J₁, OC(=X)NJ₁J₂, NJ₃C(=X)NJ₁J₂ et CN, où chaque J₁, J₂ et J₃ est, indépendamment, H ou alkyle en C₁-C₆" et X est 0, S ou NJ₁.

28. Composition de la revendication 27 dans laquelle chacun des premier et deuxième composés oligomères comprend d'environ 17 à environ 26 nucléosides et/ou monomères de longueur.

29. Procédé *in vitro* de réduction d'ARNm cible comprenant la mise en contact d'une ou plusieurs cellules ou d'un tissu avec une composition de la revendication 27 ou
une composition de la revendication 27 pour utilisation dans un procédé *in vivo* de réduction d'ARNm cible comprenant la mise en contact d'une ou plusieurs cellules, d'un tissu ou d'un animal avec une composition de la revendication 27.
